(19) Europäisches Patentamt
European Patent Office
Office européen des brevets

(11) Veröffentlichungsnummer: **0 312 895**
**A2**

(12) **EUROPÄISCHE PATENTANMELDUNG**

(21) Anmeldenummer: **88116886.8**

(22) Anmeldetag: **12.10.88**

(51) Int. Cl.4: **C07D 471/04 , C07D 495/04 , C07D 487/04 , C07D 471/14 , C07D 495/14 , C07D 403/06 , A61K 31/55 , //(C07D471/04, 243:00,221:00),(C07D495/04, 333:00,243:00),(C07D487/04, 243:00,209:00)**

(30) Priorität: **23.10.87 DE 3735895**

(43) Veröffentlichungstag der Anmeldung: **26.04.89 Patentblatt 89/17**

(84) Benannte Vertragsstaaten:
**AT BE CH DE ES FR GB GR IT LI LU NL SE**

(71) Anmelder: **Dr. Karl Thomae GmbH**
**Postfach 1755**
**D-7950 Biberach 1(DE)**

(72) Erfinder: **Eberlein, Wolfgang, Dr. Dipl.-Chem.**
**Obere Au 6**
**D-7950 Biberach 1(DE)**
Erfinder: **Engel, Wolfhard, Dr. Dipl.-Chem.**
**Mozartstrasse 13**
**D-7950 Biberach 1(DE)**
Erfinder: **Trummlitz, Günter, Dr. Dipl.-Chem.**
**Buchenweg 27**
**D-7951 Warthausen(DE)**
Erfinder: **Mihm, Gerhard, Dr. Dipl.-Chem.**
**Nickeleshalde 5/1**
**D-7950 Biberach 1(DE)**
Erfinder: **Mayer, Norbert, Dr.**
**Friedrich-Ebert-Strasse 66**
**D-7950 Biberach 1(DE)**
Erfinder: **Doods, Henri, Dr.**
**Hornsteinweg 7**
**D-7951 Warthausen 1(DE)**

(54) **Kondensierte Diazepinone, Verfahren zu ihrer Herstellung und diese Verbindungen enthaltende Arzneimittel.**

(57) Beschrieben werden neue kondensierte Diazepinone der allgemeinen Formel I

EP 0 312 895 A2

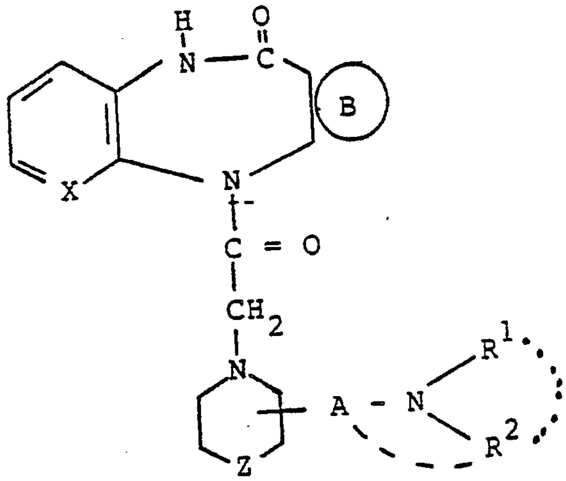

(I),

in der

]B einen der zweiwertigen Reste

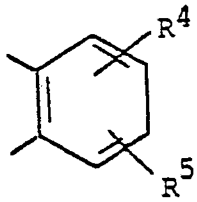

,

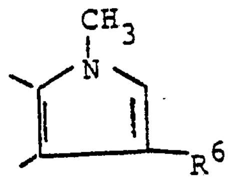

,

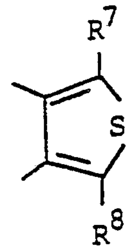

,

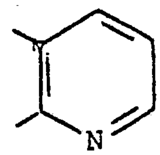

(S) (T) (U) (V)

darstellt mit den in Anspruch 1 genannten Bedeutungen für die Reste A, X, Z, $R^1$, $R^2$ und $R^4$ bis $R^8$, desweiteren ihre Salze mit anorganischen oder organischen Säuren und Verfahren zu ihrer Herstellung. Die Verbindungen zeigen günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human-und Veterinärmedizin geeignet.

## Kondensierte Diazepinone, Verfahren zu ihrer Herstellung und diese Verbindungen enthaltende Arzneimittel

Die Erfindung betrifft neue kondensierte Diazepinone, Verfahren zu ihrer Herstellung und diese Verbindung enthaltende Arzneimittel.

Aus EP-A-0 039 519 und 0 057 428 sowie aus US-A 3.660.380; 3.691.159; 4.213.984; 4.213.985; 4.210.648, 4.410.527; 4.424.225; 4.424.222 und 4.424.226 sind bereits kondensierte Diazepinone mit ulcushemmenden und magensaftsekretionshemmenden Eigenschaften bekannt.

In EP-A-0 156 191 (US-Patent 4 550 107) ist für kondensierte Diazepinone beschrieben, daß durch Einführung neuartiger Aminoacylreste gegenüber den Verbindungen der obengenannten Publikationen völlig andersartige, wertvolle pharmakologische Eigenschaften induziert werden können. Gegenüber diesen Verbindungen zeichnen sich die erfindungsgemäßen kondensierten Diazepinone überraschenderweise bei vergleichbarer oder verbesserter Selektivität durch eine wesentlich verstärkte Wirkung und Resorption nach oraler Gabe aus.

Die neuen kondensierten Diazepinone besitzen die allgemeine Formel I

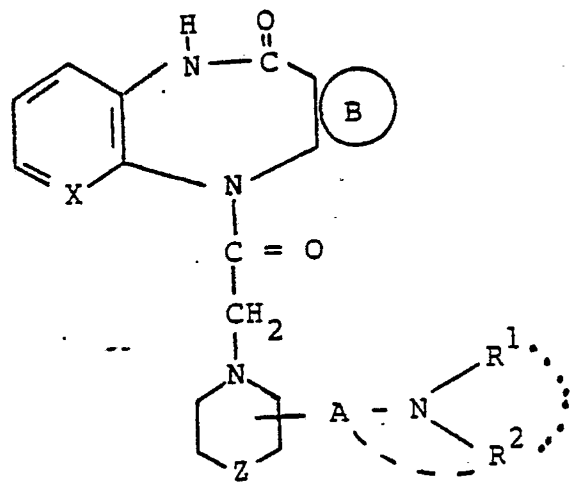

(I),

in der

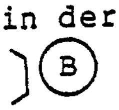 einen der zweiwertigen Reste

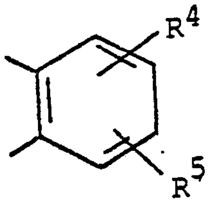

(S)

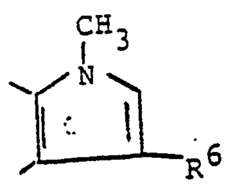

(T)

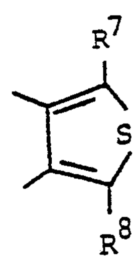

(U)

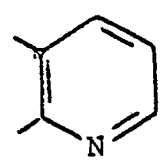

(V)

darstellt und

A, X, Z, $R^1$, $R^2$ und $R^4$ bis $R^8$ die folgenden Bedeutungen besitzen:

X ist eine =CH-Gruppe oder ein Stickstoffatom,

A ist ein geradkettiger oder verzweigter, gesättigter Alkylenrest mit 3 bis 7 Kohlenstoffatomen, der auch

durch ein Sauerstoff- oder Schwefelatom oder die Gruppe $>NR^3$ unterbrochen sein kann, worin $R^3$ eine Alkylgruppe mit 1 bis 3 Kohlenstoffatomen darstellt,

Z ist eine Einfachbindung, ein Sauerstoff- oder Schwefelatom, die Methylen- oder 1,2-Ethylengruppe,

$R^1$ ist ein verzweigter oder unverzweigter Alkylrest mit 1 bis 7 Kohlenstoffatomen, ein Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor, oder Bromatom und/oder eine Methyl-, Methoxy- oder Trifluormethylgruppe substituierte Benzoylgruppe,

$R^2$ bedeutet eine verzweigte oder unverzweigte Alkylgruppe mit 1 bis 6 Kohlenstoffatomen, $R^2$ kann aber auch ein Wasserstoffatom sein, wenn $R^1$ die vorerwähnte aliphatische Acylgruppe oder gegebenenfalls substituierte Benzoylgruppe darstellt,

$R^1$ und $R^2$ können aber auch zusammen mit dem dazwischenliegenden Stickstoffatom einen gesättigten, monocyclischen 5-, 6- oder 7-gliedrigen Ring bilden, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,

$R^2$ kann aber auch mit einem Kohlenstoffatom der A-Kette in der Weise verknüpft sein, daß zusammen mit der Gruppe $NR^1$ ein gesättigter 5-, 6- oder 7-gliedriger heterocyclischer Ring entsteht,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten.

Bevorzugte Verbindungen der obigen allgemeinen Formel I sind solche, in denen die Gruppe

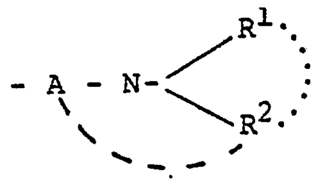

in der 3- oder 4-Stellung des gesättigten heterocyclischen Restes sitzt und

A einen geradkettigen oder verzweigten Alkylenrest mit 3 bis 5 Kohlenstoffatomen, der gegebenenfalls durch die Gruppe $>N-CH_3$ unterbrochen sein kann,

X ein Stickstoffatom oder die $=CH$-Gruppe,

] Ⓑ den zweiwertigen Rest (S) oder (U), worin $R^4$ und $R^5$ Wasserstoffatome, $R^7$ und $R^8$ Wasserstoffatome oder einer der Reste $R^4$, $R^5$, $R^7$ oder $R^8$ eine Methylgruppe darstellen,

$R^1$ und $R^2$, die gleich oder voneinander verschieden sein können, Alkylreste mit 1 bis 3 Kohlenstoffatomen oder zusammen mit dem dazwischenliegenden Stickstoffatom den 1-Piperidinylrest oder $R^2$ zusammen mit dem entsprechenden Kohlenstoffatom der Gruppe -A- den 4-Piperidinylrest der Formel

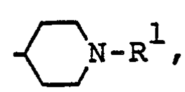

wobei $R^1$ eine niedere Alkylgruppe mit 1 bis 3 Kohlenstoffatomen darstellt und Z die Methylengruppe bedeuten.

Unter einer Cycloalkylgruppe wird beispielsweise die Cyclobutyl-, Cyclopentyl-, Cyclohexyl- oder Cycloheptylgruppe, unter einer (Cycloalkyl) alkylgruppe wird beispielsweise die Cyclopentyl- oder Cyclohexylmethyl oder -ethylgruppe verstanden. Als gegebenenfalls substituierte Aralkylgruppe für $R^1$ kommen beispielsweise in Frage: die Benzylgruppe, die 2-Phenylethylgruppe, die 3-Phenylpropylgruppe, wobei diese Gruppen in 2- oder 4-Stellung des Phenylrestes durch ein Fluor-, Chlor- oder Bromatom und/oder durch eine Methyl-, Methoxy- oder Trifluormethylgruppe substituiert sein können. Als aliphatische Acylgruppen für $R^1$ kommen insbesondere in Frage die Acetyl-, Propionyl- oder Butyrylgruppe.

Die Verbindungen der allgemeinen Formel I können nach Umsetzung mit anorganischen oder organischen Säuren auch in Form ihrer physiologisch verträglichen Salze vorliegen. Als Säuren haben sich beispielsweise Salzsäure, Bromwasserstoffsäure, Schwefelsäure, Methylschwefelsäure, Phosphorsäure,

Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Zur Erläuterung des Erfindungsgegenstandes seien als Beispiele folgende Verbindungen genannt:

5,11-Dihydro-11-[[4-[4-1-piperidinyl)butyl]-1-piperidinyl]-acetyl]-6H-pyrido[2,3-b][1,4]benzodiazepin-6-on

5,11-Dihydro-11-[[4-[3-(1-methyl-4-piperidinyl)propyl]-1-piperidinyl]acetyl]-6H-pyrido[2,3-b}[1,4]-benzodiazepin-6-on

5,11-Dihydro-11-[[4-(1-piperidinyl)propyl]-1-piperidinyl]-acetyl]-6H-pyrido[2,3-b][1,4]benzodiazepin-6-on

4,9-Dihydro-3-methyl-4-[[4-[3-(1-piperidinyl)propyl]-1-piperidinyl]acetyl]-10H-thieno[3,4-b][1,5]benzodiazepin-10-on

Erfindungsgemäß erhält man die neuen basisch substituierten kondensierten Diazepinone der allgemeinen Formel I nach folgenden Verfahren:

a) Basisch substituierte kondensierte Diazepinone der allgemeinen Formel Ia

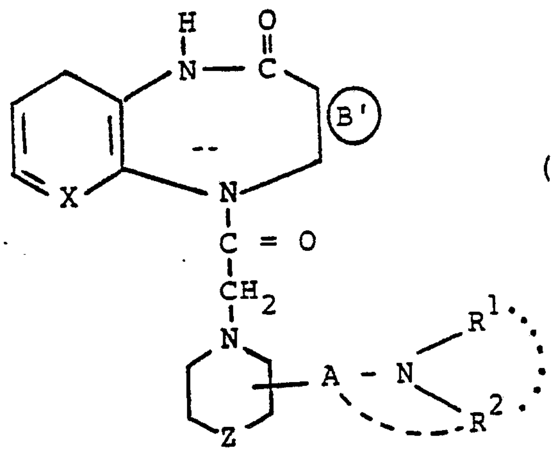

(Ia),

in der
X, Z, A und $R^1$ und $R^2$ wie oben angegeben definiert sind und ] (B') einen der zweiwertigen Reste (S), (U), (V) oder (T')

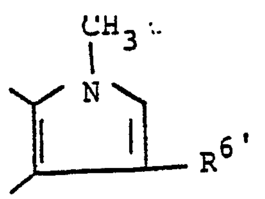

(T')

darstellt, worin $R^{6'}$ ein Chloratom oder eine Methylgruppe ist,
erhält man durch Umsetzung von Halogenacylverbindungen der allgemeinen Formel II

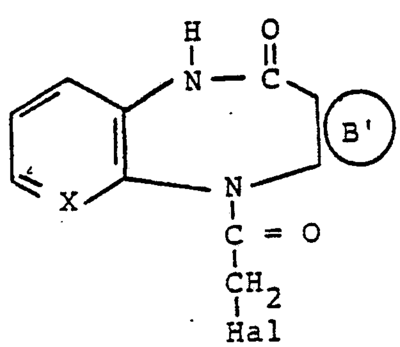

(II),

in der X und ] (B') die vorstehend genannten Bedeutungen haben und Hal ein Chlor-, Brom- oder Iodatom ist, mit sekundären Aminen der allgemeinen Formel III

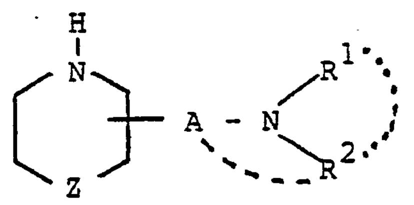

(III)

mit den für A, Z, $R^1$ und $R^2$ genannten Bedeutungen.

Die Aminierung erfolgt in einem inerten Lösungsmittel bei Temperaturen zwischen -10 °C und der Siedetemperatur des Lösungsmittels, vorzugsweise entweder mit wenigstens 2 Molen sekundärem Amin der allgemeinen Formel III oder mit 1 bis 2 Molen eines sekundären Amins der allgemeinen Formel III und einer Hilfsbase. Als Lösungsmittel kommen beispielsweise chlorierte Kohlenwasserstoffe, wie Methylenchlorid, Chloroform oder Dichlorethan; offenkettige oder cyclische Ether, wie Diethylether, Tetrahydrofuran oder Dioxan; aromatische Kohlenwasserstoffe, wie Benzol, Toluol, Xylol, Chlorbenzol oder Pyridin; Alkohole, wie Ethanol oder Isopropanol; Ketone, wie Aceton; Acetonitril, Dimethylformamid oder 1,3-Dimethyl-2-imidazolidinon in Frage. Als Hilfsbasen seien beispielsweise tertiäre organische Basen, wie Triethylamin, N-Methylpiperidin, Diethylanilin, Pyridin und 4-(Dimethylamino)pyridin oder anorganische Basen, wie Alkalimetall oder Erdalkalimetallcarbonate oder -hydrogencarbonate, -hydroxide oder -oxide genannt. Gegebenenfalls kann die Reaktion durch Zusatz von Alkalimetalliodiden beschleunigt werden. Die Reaktionszeiten betragen je nach Menge und Art des eingesetzten Amins der allgemeinen Formel III zwischen 15 Minuten und 80 Stunden.

b.) Dieselben basisch substituierten kondensierten Diazepinone der allgemeinen Formel Ia erhält man auch durch Acylierung von Diazepinonen der allgemeinen Formel IV,

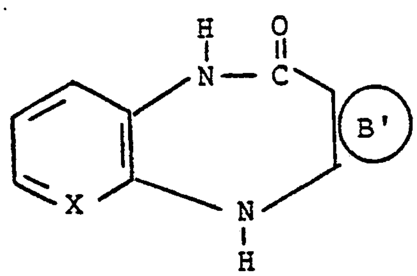

(IV),

worin X und 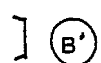 die oben angegebenen Bedeutungen haben, mit Carbonsäurederivaten der allgemeinen Formel V,

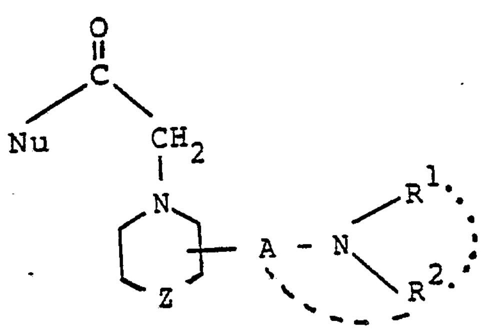

(V),

worin Z, A, $R^1$ und $R^2$ die oben angegebenen Bedeutungen haben und Nu eine nucleofuge Gruppe bzw. Abgangsgruppe darstellt.

Die Umsetzung der Verbindungen der allgemeinen Formel IV mit den Säurederivaten der allgemeinen Formel V erfolgt in an sich bekannter Weise. Die Abgangsgruppe Nu ist eine Gruppe, die gemeinsam mit der Carbonylgruppe, an die sie gebunden ist, ein reaktives Carbonsäurederivat bildet. Als reaktive Carbonsäurederivate seien beispielsweise Säurehalogenide, -ester, -anhydride oder gemischte Anhydride, wie sie aus Salzen der entsprechenden Säuren (Nu = OH) und Säurechloriden, wie Phosphoroxidchlorid, Diphosphorsäuretetrachlorid oder Chlorameisensäureestern gebildet werden oder die bei der Umsetzung von

Verbindungen der allgemeinen Formel V (Nu = OH) mit N-Alkyl-2-halogenpyridiniumsalzen entstehenden N-Alkyl-2-acyloxypyridiniumsalze genannt.

Bevorzugt wird die Reaktion mit den gemischten Anhydriden starker Mineralsäuren, insbesondere der Dichlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise Alkalimetallcarbonate oder -hydrogencarbonate, wie Natriumcarbonat oder Kaliumhydrogencarbonat; tertiäre organische Amine, wie Pyridin, Triethylamin, Ethyldiisopropylamin, 4-(Dimethylamino)pyridin, oder Natriumhydrid genannt. Die Reaktion wird bei Temperaturen zwischen -25° C und 130° C in einem inerten Lösungsmittel durchgeführt. Als inerte Lösungsmittel kommen beispielsweise chlorierte aliphatische Kohlenwasserstoffe, wie Methylenchlorid, 1,2-Dichlorethan; offenkettige oder cyclische Ether, wie Diethylether, Tetrahydrofuran oder 1,4-Dioxan; aromatische Kohlenwasserstoffe, wie Benzol, Toluol, Xylol, o-Dichlorbenzol; polare aprotische Lösungsmittel wie Acetonitril, Dimethylformamid oder Hexamethylphosphorsäuretriamid; oder Gemische davon in Frage. Die Reaktionszeiten betragen je nach Menge und Art des eingesetzten Acylierungsmittels der allgemeinen Formel V zwischen 15 Minuten und 80 Stunden. Es ist nicht nötig, die Verbindungen der allgemeinen Formel V in reiner Form herzustellen, sie können vielmehr im Reaktionsansatz in bekannter Weise in situ erzeugt werden.

c) Die unter die allgemeine Formel I fallenden neuen Pyrrolo-kondensierten Diazepinone der allgemeinen Formel Ib,

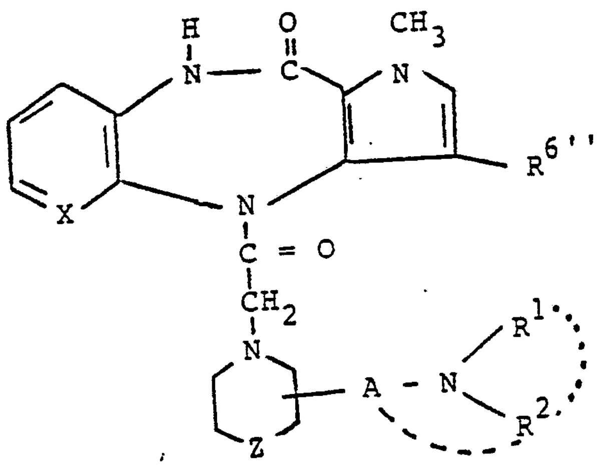

(Ib),

worin

X, Z, A, $R^1$ und $R^2$ die eingangs erwähnten Bedeutungen haben,

$R^6$ ein Wasserstoffatom darstellt, können durch Hydrogenolyse aus solchen Verbindungen der allgemeinen Formel Ia hergestellt werden, in denen ] (B′) den zweiwertigen Rest (T′) und $R^6$ ein Chloratom bedeuten.

Die Hydrogenolyse wird in Gegenwart von Katalysatoren von Metallen aus der VIII. Nebengruppe des Periodensystems der Elemente, beispielsweise von Palladium auf Tierkohle, Palladium auf Bariumsulfat, Raney-Nickel oder Raney-Cobalt, und bei Wasserstoffdrucken von 1 bis 300 bar und Temperaturen von 0° C bis 130° C in Gegenwart von Lösungsmitteln, beispielsweise Alkoholen, wie Methanol, Ethanol; Ethern wie Dioxan, Tetrahydrofuran, Carbonsäuren, beispielsweise Essigsäure oder tertiären Aminen, beispielsweise Triethylamin, durchgeführt. Arbeitet man dabei in Abwesenheit zusätzlicher Chlorwasserstoff-Akzeptoren, beispielsweise von Natriumcar bonat, Kaliumhydrogencarbonat, Triethylamin oder Natriumacetat, so entstehen direkt die Hydrochloride der gesuchten Verbindungen, die nach Entfernung des Katalysators durch Eindampfen der Reaktionslösung isoliert werden können. Ersetzt man in der vorstehenden Hydrogenolyse-Reaktion den Wasserstoff durch Ameisensäure, so gelingt die Umsetzung prinzipiell schon bei drucklosem Arbeiten. Bei dieser Variante haben sich besonders die Umsetzung mit Ameisensäure in Gegenwart von Dimethylformamid als Lösungsmittel und von Palladium auf Kohle als Katalysator bei Temperaturen zwischen 70 und 110° C, sowie die Reduktion mit Triethylammoniumformiat in Gegenwart überschüssigen Triethylamins und von Palladium auf Tierkohle oder von Palladiumacetat und Triarylphosphinen, wie Triphenylphosphin, Tris-(o-tolyl)phosphin, Tris-(2,5-diisopropylphenyl)phosphin, bei Temperaturen zwischen 40 und 110° C, bewährt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Die erfindungsgemäßen basisch substituierten kondensierten 6H-Pyrido[2,3-b][1,4]benzodiazepin-6-one der allgemeinen Formel I enthalten, insbesondere wenn ] Ⓑ den zweiwertigen Rest (U) darstellt und der Rest

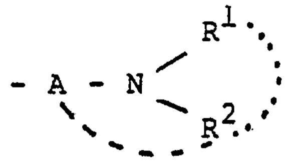

in der 2- oder 3-Stellung des gesättigten heterocyclischen Ringes sitzt, bis zu drei unabhängige chirale Elemente, davon bis zu zwei asymmetrische Kohlenstoffatome in der Seitenkette. Als weiteres chirales Element ist der acylierte Tricyclus selbst anzusehen, der in zwei spiegelbildlichen Formen vorliegen kann. Von der Natur des Tricyclus hängt es ab, ob die Energiebarriere für eine Inversion an diesem Zentrum so hoch ist, daß die einzelnen Isomeren bei Zimmertemperatur stabil sind und isolierbar werden. Es hat sich gezeigt, daß bei Verbindungen der allgemeinen Formel I, worin X ein Stickstoffatom ist und die dem Diazepinon-Ring benachbarten Positionen unsubstituiert sind, die erforderliche Aktivierungsenergie so stark vermindert ist, daß Diastereomere bei Zimmertemperatur nicht mehr nachgewiesen geschweige denn präparativ isoliert werden können.

Die erfindungsgemäßen aminoacylierten kondensierten Diazepinone der allgemeinen Formel I enthalten also bis zu drei Chiralitätselemente, von denen eines bei Zimmertemperatur unter Umständen nicht konfigurationsstabil ist. Solche Verbindungen können deshalb in mehreren disastereomeren und/oder jeweils als enantiomere (+)- und (-)-Formen auftreten. Die Erfindung umfaßt die einzelnen Isomeren ebenso wie ihre Gemische. Die Trennung der jeweiligen Diastereomeren gelingt auf Grund der unterschiedlichen physiko-chemischen Eigenschaften, z.B. durch fraktioniertes Umkristallisieren aus geeigneten Lösungsmitteln, durch Hochdruckflüssigkeitschromatographie, Säulenchromatographie oder gaschromatographische Verfahren.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (-)-Weinsäure, oder eines Derivats davon, wie (+)- oder (-)-Diacetylweinsäure, (+)- oder (-)-Monomethyltartrat oder (+)-Camphersulfonsäure.

Nach einem üblichen Verfahren zur Isomerentrennung wird das Racemat einer Verbindung der allgemeinen Formel I mit einer der vorstehend angegebenen optisch aktiven Säuren in äquimolarer Menge in einem Lösungsmittel umgesetzt und die erhaltenen kristallinen diastereomeren Salze werden unter Ausnut zung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemische, beispielsweise im Volumenverhältnis 50:50, verwendet. Sodann wird jedes der diastereomeren Salze in Wasser gelöst, mit einer Base, wie Natriumcarbonat oder Kaliumcarbonat, neutralisiert und dadurch die entsprechende freie Verbindung in der (+)- oder (-)-Form erhalten.

Jeweils nur ein Enantiomeres bzw. ein Gemisch zweier optisch aktiver unter die allgemeine Formel I fallender diastereomerer Verbindungen wird auch dadurch erhalten, daß man die oben beschriebenen Synthesen mit nur einem Enantiomeren der allgemeinen Formel III bzw. V durchführt.

Zur Herstellung der Halogenacylverbindungen der allgemeinen Formel II werden die Ausgangsverbindungen der allgemeinen Formel IV

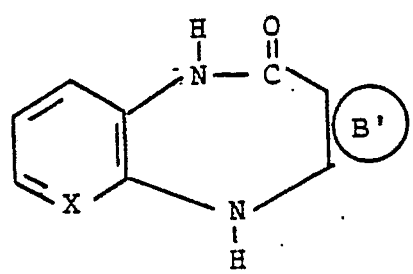

(IV),

mit Verbindungen der allgemeinen Formel $Hal\text{-}CH_2CO\text{-}Hal'$ (VII) oder $[Hal\text{-}CH_2\text{-}CO]_2O$ (VIII), worin Hal′ eine

der Bedeutungen von Hal hat und Hal wie oben definiert ist, umgesetzt. Diese Acylierung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide der allgemeinen Formel VII sind gegenüber den Säureanhydriden der allgemeinen Formel VIII bevorzugt. Als Säurehalogenid der allgemeinen Formel VII ist Chloracetylchlorid, als Säureanhydrid der allgemeinen Formel VIII ist Chloressigsäureanhydrid bevorzugt. Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid. Als Hilfsbasen seien z.B. tertiäre organische Basen, wie Triethylamin und Ethyldiisopropylamin, oder Pyridin; oder anorganische Basen, wie wasserfreie Alkalimetall- oder Erdalkalimetallcarbonate oder -hydrogencarbonate oder Erdalkalimetalloxide genannt. Als Acylierungskatalysatoren kommen beispielsweise in Frage Imidazol, Pyridin oder 4-Dimethylaminopyridin.

Bedeutet in einer Verbindung der allgemeinen Formel II Hal ein Chloratom, so kann es durch Umsetzung mit Natriumiodid in Aceton oder Ethanol leicht gegen das reaktivere Iod ausgetauscht werden (in diesem Zusammenhang sei auch auf das US-Patent Nr. 4 550 107 verwiesen).

Zwischenverbindungen der allgemeinen Formel III, die einen in $\beta$-Stellung zum gesättigten Heterocyclus durch ein Heteroatom unterbrochenen Alkylenrest A besitzen, können in Analogie zu im DE-A-36 26 095 ausführlich diskutierten Methoden synthetisiert werden.

Zwischenverbindungen der allgemeinen Formel III, in der Z eine Methylengruppe bedeutet, bereitet man zweckmäßig aus entsprechend substituierten Pyridinen, beispielsweise durch katalytische Hydrierung in ethanolisch-salzsaurer Lösung und unter Verwendung von Platin(IV)-oxid als Katalysator (siehe auch: F.F. Blicke u.a., J. Org. Chemistry 26, 3258 (1961)) oder in Eisessig und in Gegenwart von Platin(IV)oxid (siehe auch W.F. Minor u.a., J. Med. Pharm. Chem. 5, 96, 105 ff. (1962) und A.H. Sommers u.a., J. Amer. Chem. Soc. 75, 57, 58 ff. (1953)). Die substituierten Pyridine ihrerseits lassen sich nach dem Fachmann geläufigen Methoden leicht synthetisieren, z. B. durch Addition entsprechender sekundärer Amine, Dialkylaminoalkanole oder Dialkylaminoalkanthiole an Vinylpyridine, durch Reduktion von geeigneten Pyridinalkansäureamiden mit Lithiumaluminiumhydrid, durch Alkylierung von Picolinen mit Dialkylaminoalkylhalogeniden in Gegenwart von Lithiumdiisopropylamid oder Natriumamid (siehe auch A.E. Tschitschibabin, Bull. Soc. Chim. France 1938, 436) oder auch durch Umsetzung von ($\omega$-Halogenalkyl)-pyridinen mit Dialkylaminoalkanolen, Dialkylaminoalkanthiolen oder sekundären Aminen (siehe auch L. Rondahl, Acta Pharm. Suec. 13, 229-34 (1976)) bzw. deren metallierten Derivaten.

Ein allgemein anwendbares Verfahren zur Synthese von Aminen der allgemeinen Formel III besteht in der Reduktion geeigneter heterocyclisch substituierter und gegebenenfalls im Alkylenrest durch Heteroatome unterbrochener Alkancarbonsäuredialkylamide, beispielsweise durch Lithiumaluminiumhydrid. Von den Vorstufen her gegebenenfalls an der Stickstoffunktion des gesättigten Heterocyclus noch vorhandene Schutzgruppen können abschließend in üblicher Weise abgespalten werden, ein Benzylrest z. B. durch Hydrogenolyse in Gegenwart von Palladium/Tierkohle. Beispielsweise kann man 5-Oxo-2-pyrrolidinessigsäure (G.L. Evans u.a., J. Amer. Chem. Soc., 72 2727 (1950)) nacheinander mit Thionylchlorid und einem interessierenden Dialkylamin umsetzen und das so hergestellte N,N-Dialkyl-5-oxo-2-pyrrolidinacetamid anschließend mit Lithiumaluminiumhydrid zum gewünschten 2-[2-(Dialkylamino)- ethyl]pyrrolidin reduzieren; oder man kann das aus 4-Benzyl-3-(hydroxymethyl)-morpholin (G.R. Brown u.a., J. Chem. Soc. Perkin Trans. I 1985, 2577) durch Einwirkung von Thionylchlorid erhältliche 4-Benzyl-3-(chlormethyl)-morpholin-hydrochlorid durch Kettenverlängerung in üblicher Weise in (4-Benzyl-3-morpholinyl)alkansäuren überführen und somit zur Synthese von 3-(Dialkylaminoalkyl)morpholinen heranziehen.

Die Verbindungen der allgemeinen Formel III, worin $R^2$ mit dem entsprechenden Kohlenstoffatom der Gruppe -A- zu einem 4-Piperidinylrest verknüpft ist, erhält man beispielsweise durch Umsetzung einer Verbindung der allgemeinen Formel

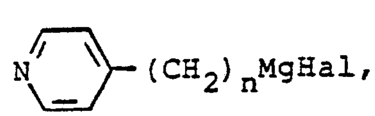

worin n die Zahl 1 bis 4 darstellt, mit einer Verbindung der allgemeinen Formel

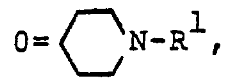

worin $R^1$ wie oben definiert ist, wobei nach Abspaltung von Wasser eines der beiden Isomeren der allgemeinen Formel

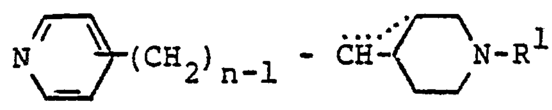

oder deren Gemisch entsteht, das anschließend in Gegenwart von Platindioxid als Katalysator in Essigsäure zum gesuchten Diamin hydriert wird.

Die Ausgangsverbindungen der allgemeinen Formel V, in der Nu eine Alkoxygruppe bedeutet, erhält man durch Umsetzung von Diaminen der allgemeinen Formel III mit Halogenessigsäureestern, gegebenenfalls unter Verwendung zusätzlicher Hilfsbasen, z.B. Triethylamin, oder Katalysatoren, beispielsweise Triton B. Durch Verseifung der erhaltenen Ester, z.B. mit Barytlauge, erhält man die unter die allgemeine Formel V fallenden Carbonsäuren, die zur Herstellung von Derivaten mit anderen nucleofugen Gruppen dienen können.

Ein weiterer Gegenstand der Erfindung sind Arzneimittel, die ein oder mehrere kondensierte Diazepinone der allgemeinen Formel I bzw. deren physiologisch verträgliche Salze enthalten.

Die Verbindungen der allgemeinen Formel I lassen sich hierzu in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die basisch substituierten kondensierten Diazepinone der allgemeinen Formel I und ihre Säureadditionssalze besitzen wertvolle Eigenschaften; insbesondere besitzen sie günstige Effekte auf die Herzfrequenz und sind angesichts fehlender magensäuresekretionshemmender, salivationshemmender und mydriatischer Einflüsse als vagale Schrittmacher zur Behandlung von Bradycardien und Bradyarrhythmien in der Human- und auch der Veterinärmedizin geeignet; ein Teil der Verbindungen zeigt auch spasmolytische Eigenschaften auf periphere Organe, insbesondere Colon und Blase.

Eine günstige Relation zwischen tachycarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

A Bindungsstudien an muscarinischen Rezeptoren: Bestimmung des $IC_{50}$-Wertes in vitro

Als Organspender dienten männliche Sprague-Dawley-Ratten mit 180-220 g Körpergewicht. Nach Entnahme von Herz, Submandibularis und Großhirnrinde wurden alle weiteren Schritte in eiskaltem Hepes-HCl-Puffer (pH 7,4; 100 m molar NaCl, 10 m molar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1: 400 |
| Großhirnrinde | 1:3000 |
| Submandibularis | 1: 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 n molar $^3H$-N-Methylscopolamin ($^3H$-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3H$-NMS. Der Anteil der unspezifischen Bindung wurde definiert als jene Radioaktivität, die in Anwesenheit von 1 µ molar Quinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie re präsentieren jene Konzentration der Testsubstanz, bei welcher die spezifische Bindung von $^3H$-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird eine Beschreibung von Methoden wiedergegeben, die zur Erfassung von cardioselektiven Antimuscarinica geeignet sind.

"In vivo" Methoden

Die angewandten Methoden hatten zum Ziel, die Selektivität der antimuscarinischen Wirkung zu bestätigen. Jene Substanzen, die auf der Basis von "in vitro" Untersuchungen ausgewählt worden waren, wurden auf ihre

1. $M_1$-/$M_2$-Selektivität an der Ratte
2. Speichselsekretionshemmende Wirkung an der Ratte
3. Hemmung der Acetylcholinwirkung auf Blase, Bronchien und Herzfrequenz des Meerschweinchens

untersucht.

1. $M_1$-/$M_2$-Selektivität an der Ratte

Die angewandte Methode wurde von Hammer und Giachetti (Life Sciences 31, 2991-2998 (1982)) beschrieben. 5 Minuten nach intravenöser Injektion steigender Dosen der Substanz wurden entweder der rechte Vagus elektrisch stimuliert (Frequenz: 25 Hz; Pulsbreite: 2ms; Reizdauer: 30s; Voltzahl: supramaximal) oder 0,3 mg/kg McN-A-343 in männliche THOM-Ratten intravenös injiziert. Die durch Vagusstimulation hervorgerufene Bradykardie und der durch McN-A-343 verursachte Blutdruckanstieg wurden bestimmt. Die Dosis der Substanzen, die entweder die vagale Bradykardie ($M_2$) oder den Blutdruckanstieg ($M_1$) um 50% verminderte, wurde graphisch ermittelt. Ergebnisse siehe Tabelle II.

2. Speichselsekretionshemmende Wirkung an der Ratte

Nach Lavy und Mulder (Arch. int. Pharmacodyn. 178, 437-445, (1969)) erhielten mit 1,2 g/kg Urethan narkotisierte männliche THOM-Ratten steigende Dosen der Substanz i.v.. Die Speichelsekretion wurde durch s.c. Gabe von 2 mg/kg Pilocarpin ausgelöst. Der Speichel wurde mit Fließpapier aufgesaugt, die von ihm eingenommene Fläche alle 5 Minuten planimetrisch bestimmt. Die Dosis der Substanz, die das Speichelvolumen um 50% verminderte, wurde graphisch ermittelt. Ergebnisse siehe Tabelle II.

3. Hemmung der Acetylcholinwirkung auf Blase, Bronchien und Herzfrequenz des Meerschweinchens

Am narkotisierten Meerschweinchen wurden 5 Minuten nach Gabe der Prüfsubstanz 10 µg/kg Acetylcholin intravenös als auch gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus -log $ED_{50}$-Werte bestimmt. Ergebnisse siehe Tabelle III.

Nach den vorstehenden Angaben wurden beispielsweise die folgenden Verbindungen untersucht:

A = 4,9-Dihydro-3-methyl-4-[[4-[3-(1-piperidinyl)propyl]-1-piperidinyl]acetyl]-10H-thieno[3,4-b][1.5]-benzodiazepin-10-on,

B = 5,11-Dihydro-11-[[4-(3-(1-piperidinyl)propyl]-1-piperidinyl]acetyl]-6H-pyrido[2,3-b][1,4]-benzodiazepin-6-on,

C = 5,11-Dihydro-11-[[4-[4-(1-piperidinyl)butyl]-1-piperidinyl]acetyl]-6H-pyrido[2,3-b][1,4]-benzodiazepin-6-on,

D = 5,11-Dihydro-11-[[4-[3-(1-methyl-4-piperidinyl)propyl]-1-piperidinyl]acetyl]-6H-pyrido[2,3-b][1,4]-benzodiazepin-6-on,

und als Vergleichssubstanzen

E = 11-[[2-[(Diethylamino)methyl]-1-piperidinyl]acetyl]-5,11-dihydro-6H-pyrido[2,3-b][1,4]-benzodiazepin-6-on (siehe US-Patent Nr. 4 550 107)

F = 5,11-Dihydro-11-[(4-methyl-1-piperazinyl)acetyl]-6H-pyrido[2,3-b][1,4]benzodiazepin-6-on (Pirenzepin, siehe US-Patent Nr. 3 660 380)

und

G = Atropin

Tabelle I:

| Rezeptor-Bindungs-Tests, in vitro: | | | |
|---|---|---|---|
| Ergebnisse: | | | |
| Substanz | Rezeptor-Bindungs-Tests $IC_{50}[nMl^{-1}]$ | | Submandibularis |
| | Cortex | Herz | |
| A | 60 | 5 | 200 |
| B | 200 | 20 | 550 |
| C | 50 | 9 | 200 |
| D | 500 | 50 | 1500 |
| E | 1200 | 140 | 5000 |
| F | 100 | 1500 | 200 |
| G | 2 | 4 | 4 |

Die Angaben in der vorstehenden Tabelle I beweisen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus dem Herzen gegenüber solchen aus der Großhirnrinde und Submandibularis.

Tabelle II:

| $M_1$-/$M_2$-Selektivität und Speichelsekretionshemmung an der Ratte: | | | |
|---|---|---|---|
| Ergebnisse: | | | |
| Substanz | -log $ED_{50}$[$Molkg^{-1}$] | | Salivation |
| | Herz | Blutdruck | |
| A | 7,14 | 5,82 | 5,20 |
| B | | | |
| C | 6,94 | 5,42 | < 4,5 |
| D | | | |
| E | 6,42 | 5,63 | 5,00 |
| F | 5,60 | 6,94 | 6,22 |
| G | 7,94 | 7,34 | 7,60 |

Tabelle III:

| Hemmung der Acetylcholinwirkung auf Blase, Brochien und Herzfrequenz des Meerschweinchens: | | | |
|---|---|---|---|
| Ergebnisse: | | | |
| Substanz | -log $ED_{50}$[$Molkg^{-1}$] | | Blase |
| | Herz | Bronchien | |
| A | 6,91 | 6,55 | 5,56 |
| B | 6,91 | 6,33 | 5,74 |
| C | 7,06 | 6,18 | 5,58 |
| D | 6,45 | 6,07 | < 5,0 |
| E | 5,84 | 5,58 | 4,73 |
| F | 5,85 | 6,57 | 5,36 |
| G | 7,70 | 7,96 | 7,03 |

Aus den pharmakologischen Daten der vorstehenden Tabellen II und III ergibt sich - in völliger Übereinstimmung mit den Rezeptor-Bindungs-Studien -, daß die Herzfrequenz durch die genannten Verbindungen bereits bei Dosierungen gesteigert wird, bei denen noch keine Einschränkung der Speichselsekretion beobachtet wird.

Außerdem deuten die pharmakologischen Daten der vorstehenden Tabelle III auf ein überraschend großes Unterscheidungsvermögen zwischen Herz und glatter Muskulatur.

Die genannten Substanzen weisen gegenüber der bereits bekannten Verbindung E eine wesentlich gesteigerte Wirkungsstärke auf. Dabei bleibt die therapeutisch nutzbare Selektivität erhalten. Dies führt zu einer geringeren Substanzbelastung des Patienten, ohne das Risiko muskarinischer Nebenwirkungen zu erhöhen.

Ferner sind die erfindungsgemäß hergestellten Verbindungen gut verträglich, so konnten selbst bei den höchsten applizierten Dosen bei den pharmakologischen Untersuchungen keine toxischen Nebenwirkungen beobachtet werden.

Die nachfolgenden Beispiele sollen die Erfindung näher erläutern:

Beispiel 1

5,11-Dihydro-11-[[4-[4-(1-piperidinyl)butyl]-1-piperidinyl]-acetyl]-6H-pyrido[2,3-b][1,4]benzodiazepin-6-on

Ein Gemisch aus 5,32 g (0,018 Mol) 11-(Chloracetyl)-5,11-dihydro-6H-pyrido[2,3-b][1,4]benzodiazepin-6-on, 150 ml Acetonitril, 4,02 g (0,018 Mol) 4-[4-(1-Piperidinyl)butyl]piperidin und 1,93 g (0,019 Mol) Triethylamin wird drei Stunden unter Rückfluß und Rühren erhitzt. Nach dem Abkühlen engt man im Vakuum zur Trockne ein. Der Rückstand wird in gesättigter Kaliumcarbonat-Lösung digeriert, wobei sich zwei Phasen bilden. Man trennt die organische Phase im Scheidetrichter ab und extrahiert die wässrige Phase mehrfach mit Essigester. Die vereinigten organischen Extrakte werden mehrfach mit gesättigter Natriumchlorid-Lösung ausgewaschen, über Aktivkohle filtriert und nach dem Trocknen über Natriumsulfat im Vakuum eingedampft. Der Rückstand wird an Kieselgel unter Verwendung einer Mischung von Methylenchlorid/Cyclohexan/Methanol/Essigester/Ammoniak (750:57:57:195:7,5 v:v:v:v:v) als Elutionsmittel chromatographiert.

Nach dem Eindampfen der entsprechenden Fraktionen erhält man einen Eluatrückstand, der aus Essigester/Ethanol umkristallisiert wird. Man erhält farblose Kristalle vom Fp. 228-229 °C.
Ausbeute: 3,8 g (45 % der Theorie).

Beispiel 2

5,11-Dihydro-11-[[4-[3-(1-methyl-4-piperidinyl)propyl]-1-piperidinyl]acetyl]-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 4-[3-(1-Methyl-4-piperidinyl)propyl]piperidin, jedoch unter Verwendung von Dimethylformamid an Stelle von Acetonitril als Lösungsmittel, in einer Ausbeute von 20 % der Theorie.
Farblose Kristalle vom Fp. 192-194 °C (Isopropanol/Diisopropylether).

Beispiel 3

5,11-Dihydro-11-[[4-[3-(1-piperidinyl)propyl]-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 4-[3-(1-Piperidinyl)propyl]piperidin in einer Ausbeute von 87 % der Theorie.
Farblose Kristalle vom Fp. 202-203 °C (Essigester).

Beispiel 4

6,11-Dihydro-11-[[4-[4-(1-piperidinyl)butyl]-1-piperidinyl]acetyl]-5H-pyrido[2,3-b][1,5]benzodiazepin-5-on

Hergestellt analog Beispiel 1 aus 11-(Chloracetyl)-6,11-dihydro-5H-pyrido[2,3-b][1,5]benzodiazepin-5-on und 4-[4-(1- Piperidinyl)butyl]piperidin in einer Ausbeute von 37 % der Theorie.
Farblose Kristalle vom Fp. 167-168 °C.

| $C_{28}H_{37}N_5O_2$ (475,64) | | | | | | |
|---|---|---|---|---|---|---|
| Ber.: | C | 70,71 | H | 7,84 | N | 14,72 |
| Gef.: | | 70,44 | | 7,75 | | 14,65 |

Beispiel 5

11-[[4-[4-(Diethylamino)butyl-1-piperidinyl]acetyl]-6,11-dihydro-5H-pyrido[2,3-b][1,5]benzodiazepin-5-on

Hergestellt analog Beispiel 1 aus 11-(Chloracetyl)-6,11-dihydro-5H-pyrido[2,3-b][1,5]benzodiazepin-5-on und 4-[4-(Diethylamino)butyl]piperidin in einer Ausbeute von 24 % der Theorie.
Farblose Kristalle vom Fp. 147-148 °C (Essigester).

| $C_{27}H_{37}N_5O_2$ (463,63) | | | | | | |
|---|---|---|---|---|---|---|
| Ber.: | C | 69,95 | H | 8,04 | N | 15,11 |
| Gef.: | | 69,62 | | 7,89 | | 15,18 |

Beispiel 6

4-[[4-(4-Diethylamino)butyl]-1-piperidinyl]acetyl]-4,9-dihydro-3-methyl-10H-thieno[3,4-b][1,5]benzodiazepin-10-on

Hergestellt analog Beispiel 1 aus 4-(Chloracetyl)-4,9-dihydro-3-methyl-10H-thieno[3,4-b][1,5]-benzodiazepin-10-on und 4-[4-(Diethylamino)butyl]piperidin in einer Ausbeute von 58 % der Theorie.
Farblose Kristalle vom Fp. 192-194 °C.

Beispiel 7

11-[[2[3-(Diethylamino)propyl]-1-piperidinyl]acetyl]-5,11-dihydro-6H-pyrido[2,3-b][1,4]benzodiazepin-6-on

Hergestellt analog Beispiel 1 aus 11-(Chloracetyl)-5,11-di-hydro-6H-pyrido[2,3-b][1,4]benzodiazepin-6-on und 2-[3-(Diethylamino)propyl}piperidin in einer Ausbeute von 74 % der Theorie.
Farblose Kristalle vom Fp. 151-153 °C (Acetonitril).

Beispiel 8

5,11-Dihydro-11-[[3-[3-[4-(aminocarbonyl)-1-piperidinyl]propyl]-1-piperidinyl]acetyl]-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 3-[3-[4-(aminocarbonyl)-1-piperidinyl]propyl]piperidin, jedoch unter Verwendung von Dimethylformamid an Stelle von Acetonitril als Lösungsmittel, in einer Ausbeute von 33 % der Theorie.
IR ($CH_2Cl_2$): 1670 $cm^1$ (C=O); $^1H$-NMR ($CDCl_3/CD_3OD$, 400 MHz):
δ: 8,30 (br.,s, 1H); 7,95 (br.,s, 1H); 7,65 (m, 3H); 7,50 (m, 1H); 7,40 (m, 1H); 3,65-3,35 (m, 2H); 3,30-2,90 (m, 3H); 2,75 (br.,s, 1H); 2,50-2,15 (m, 4H); 2,15-1,30 (m, 13H; 1,10 (br.,s, 3H); 0,75 (m, 1H).
MS: $M^+$ 504 m/e (Molekulargewicht berechnet 504,644 g/mol).

Beispiel 9

5,11-Dihydro-11-[[2-[3-(dimethylamino)propyl]-1-piperidinyl]acetyl]-6H-pyrido[2,3-b][1,4]benzodiazepin-6-on

Hergestellt analog Beispiel 1 aus 11-(Chloracetyl]-5,11-dihydro-6H-pyrido[2,3-b][1,4]benzodiazepin-6-on

und 2-[3-(Dimethylamino)propyl]piperidin in einer Ausbeute von 25 % der Theorie.
Farblose Kristalle vom Fp. 174-176 °C (Acetonitril).

Beispiel 10

11-[[2-[4-(Diethylamino)butyl]-1-piperidinyl]acetyl]-5,11-dihydro-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 2-[4-(Diethylamino)butyl]piperidin in einer Ausbeute von 54 % der Theorie.
Farblose Kristalle vom Fp. 149-150 °C (Essigester).

Beispiel 11

5,11-Dihydro-11-[[2-[4-(dimethylamino)butyl]-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 2-[4-(Dimethylamino)butyl]piperidin in einer Ausbeute von 25 % der Theorie.
Farblose Kristalle vom Fp. 157-159 °C (Essigester)

Beispiel 12

5,11-Dihydro-11-[[3-[3-(dimethylamino)propyl]-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 3-[3-(Dimethylamino)propyl]piperidin in einer Ausbeute von 7 % der Theorie.
$R_F$ 0,35 (Merck DC-Fertigplatten, Kieselgel $F_{254}$;
Fließmittel: Dichlormethan/Methanol/konz. Ammoniak 90/10/1)
IR ($CH_2Cl_2$): 1665 $cm^{-1}$ (C=O), 1680 $cm^{-1}$(C=O);
$^1$H-NMR (DMSO-$d_6$/$CD_3OD$, 80 MHz):
$\delta$: 8,2 (d, 1H); 8,0-7,2 (m, 6H); 4,0-2,2 (m,~8H); 2,15 (d, 6H, $N(CH_3)_2$); 2,0-0,6 (m,~9H).

Beispiel 13

5,11-Dihydro-11-[[4-(1-methyl-4-piperidinyl)-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 4-(1-Methyl-4-piperidinyl)piperidin in einer Ausbeute von 23 % der Theorie.
Farblose Kristalle vom Fp. 208-211 °C (Essigester).

Beispiel 14

5,11-Dihydro-11-[[2-(1-methyl-4-piperidinyl)-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 2-(1-Methyl-4-piperidinyl]piperidin in einer Ausbeute von 27 % der Theorie.
Farblose Kristalle vom Fp. 172-173 °C (Diisopropylether).

Beispiel 15

5,11-Dihydro-11-[[2-[4-(1-piperidinyl)butyl]-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 2-[4-(1-Piperidinyl)butyl]piperidin in einer Ausbeute von 42 % der Theorie.
Farblose Kristalle vom Fp. 144-145 °C (Acetonitril).

Beispiel 16

11-[[3-[4-[(Benzoyl)methylamino]butyl]-1-piperidinyl]acetyl]-5,11-dihydro-6H-pyrido[2,3-b][1,4]-benzodiazepin-6-on-hydrochlorid

Hergestellt analog Beispiel 1 aus 11-(Chloracetyl)-5,11-dihydro-6H-pyrido[2,3-b][1,4]benzodiazepin-6-on und 3-[4-[(Benzoyl)methylamino]butyl]piperidin. Durch Auflösen der Base in Essigester und Zugabe von etherischer Salzsäure wurde das Hydrochlorid in einer Ausbeute von 62 % erhalten. IR ($CH_2Cl_2$): 1630 $cm^{-1}$; 1670-1700 $cm^{-1}$ (C=O);
$^1$H-NMR (DMSO-$d_6$; 400 MHz):
δ: 11,1 (s, 1H); 8,35 (d,1H); 8,0-7,3 (m, 11H); 4,7-4,4 (m, 1H), 4,1-3,9 (br.,s, 1H); 3,8-2,5 (m, ~10H); 2,0-0,8 (m, ~10H).

Beispiel 17

5,11-Dihydro-11-[[3-[4-[(2-phenylethyl)methylamino]butyl]-1-piperidinyl]acetyl]-6H-pyrido[2,3-b][1,4]-benzodiazepin-6-on

Hergestellt analog Beispiel 1, jedoch unter Verwendung von Dimethylformamid an Stelle von Acetonitril als Lösungsmittel, aus 11-(Chloracetyl)-5,11-dihydro-6H-pyrido[2,3-b][1,4]benzodiazepin-6-on und 3-[4-[(2-Phenylethyl)methylamino]butyl]piperidin in einer Ausbeute von 46 % der Theorie.
Farblose Kristalle vom Fp. 154-155 °C (Essigester).

Beispiel 18

11-[[3-[4-[(Acetyl)methylamino]butyl]-1-piperidinyl]acetyl]-5,11-dihydro-6H-pyrido[2,3-b][1,4]benzodiazepin-6-on-hydrochlorid

Hergestellt analog Beispiel 1 aus 11-(Chloracetyl)-5,11-dihydro-6H-pyrido[2,3-b][1,4]benzodiazepin-6-on und 3-[4-[(Acetyl)methylamino]butyl]piperidin, jedoch unter Verwendung von Dimethylformamid an Stelle von Acetonitril als Lösungsmittel. Durch Auflösen der Base in Essigester und Zu gabe von etherischer Chlorwasserstofflösung wurde das Hydrochlorid in einer Ausbeute von 73 % der Theorie erhalten.
IR ($CH_2Cl_2$): 1660 $cm^{-1}$ (C=O);
$^1$H-NMR (DMSO-$d_6$/$CD_3OD$; 80 MHz):
δ: 8,3 (d, 1H); 7,9-7,3 (m, 6H); 4,2-2,5 (m, 10H); 2,1-1,0 (m, 14H).
MS: $M^+$: 463 m/e (entspr. $C_{26}H_{33}N_5O_3$).

Beispiel 19

11-[[3-[3-[(Acetyl)methylamino]propyl]-1-piperidinyl]acetyl]-5,11-dihydro-6H-pyrido[2,3-b][1,4]benzodiazepin-6-on

Hergestellt analog Beispiel 1 aus 11-(Chloracetyl]-5,11-dihydro-6H-pyrido[2,3-b][1,4]benzodiazepin-6-on und 3-[3-[(Acetyl)methylamino]propyl]piperidin, jedoch unter Verwendung von Dimethylformamid an Stelle von Acetonitril als Lösungsmittel, in einer Ausbeute von 11 % der Theorie.
Farblose Kristalle vom Fp. 170-172 °C (Essigester).

Beispiel 20

11-[[3-[3-[(Benzoyl)methylamino]propyl]-1-piperidinyl]acetyl]-5,11-dihydro-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 3-[3-[(Benzoyl)methylamino]propyl]piperidin in einer Ausbeute von 61 % der Theorie.
Farblose Kristalle vom Fp. 172-175 °C (Essigester).

Beispiel 21

5,11-Dihydro-11-[[3-[3-[[2-(dimethylamino)ethyl]methylamino]propyl]-1-piperidinyl]acetyl]-6H-pyrido[2,3-b]-[1,4]-benzodiazepin-6-on

Hergestellt analog Beispiel 1 aus 11-(Chloracetyl)-5,11-dihydro-6H-pyrido[2,3-b][1,4]benzodiazepin-6-on und 3-[3-[[2-(Dimethylamino)ethyl]methylamino]propyl]piperidin, jedoch unter Verwendung von Dimethylformamid an Stelle von Acetonitril als Lösungsmittel, in einer Ausbeute von 57 % der Theorie.
$R_F$ = 0,3 (Methylenchlorid/Methanol/ Ammoniak = 50:10:1, v:v:v;
Merck-DC-Fertigplatten Kieselgel F-254).

IR $CH_2Cl_2$): 3370 $cm^{-1}$ (N-H)
1680 $cm^{-1}$, 1665 $cm^{-1}$ (C=O)

$^1$H- NMR ($CDCl_3$/$CD_3OD$; 400 MHz):
δ: 8,4-7,3 (5H, aromat. H); 3,7-3,0 (2H, N-CO-$CH_2$-N); 3,0-2,1 (17H); 2,0-0,6 (13H).
MS: $M^+$: 478 m/e, berechnetes Molekulargewicht: 478,6 g/mol.

Beispiel 22

11-[[3-[3-[3-(>Diethylaminocarbonyl)-1-piperidinyl]propyl]-1-piperidinyl]acetyl]-5,11-dihydro-6H-pyrido[2,3-b]-[1,4]-benzodiazepin-6-on

Hergestellt analog Beispiel 1 aus 11-(Chloracetyl)-5,11-dihydro-6H-pyrido[2,3-b][1,4]benzodiazepin-6-on und 3-[3-[3-(Diethylaminocarbonyl)-1-piperidinyl]propyl]piperidin, jedoch unter Verwendung von Dimethylformamid an Stelle von Acetonitril als Lösungsmittel, in einer Ausbeute von 64 % der Theorie.
IR ($CH_2Cl_2$): 1665, 1675 $cm^{-1}$ (C=O)
MS: $M^+$ 478.

Beispiel 23

5,11-Dihydro-11-[[3-[3-(1-piperidinyl)propyl]-1-piperidinyl]acetyl]-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 3-[3-(1-Piperidinyl)propyl]piperidin, jedoch unter Verwendung von Dimethylformamid an Stelle von Acetonitril als Lösungsmittel, in einer Ausbeute von 42 % der Theorie.
Farblose Kristalle vom Fp. 192-194 °C (Diisopropylether).

Beispiel 24

5,11-Dihydro-11-[[4-[3-(1-piperidinyl)propyl]-1-piperidinyl]acetyl]-6H-pyrido[2,3-b][1,4]benzodiazepin-6-on

Man erwärmt ein Gemisch von 16,90 g (0,063 Mol) 4-[3(1-Piperidinyl)propyl]piperidinessigsäure und 2,0 g einer 75 proz. Natriumhydrid-Dispersion in Paraffinöl in 160 ml Dimethylformamid bei 50 bis 80 °C so lange bis die Wasserstoffentwicklung beendet ist. Zu dem entstandenen Natriumsalz der genannten Säure fügt man 13,0 g (0,062 Mol) 5,11-Dihydro-6H-pyrido[2,3-b][1,4]benzodiazepin-6-on zu und tropft bei -10 °C 9,8 g (0,064 Mol) Phosphoroxidchlorid innerhalb von 10 Minuten zu. Man rührt 4 Stunden bei -10 °C, 4 Stunden bei 0 °C und 20 Stunden bei Zimmertemperatur. Anschließend rührt man die Mischung in 300 g Eis eine, stellt mit Natronlauge auf pH 9 und schüttelt erschöpfend mit Dichlormethan aus. Die vereinigten organischen Phasen werden einmal mit wenig Eiswasser gewaschen , über Natriumsulfat getrocknet und eingedampft. Der Rückstand wird aus Essigester unter Verwendung von Aktivkohle umkristallisiert. Farblose Kristalle vom Fp. 202-203 °C, nach Dünnschichtchromatogramm, Mischschmelzpunkt, IR-, UV- und $^1$H-NMR-Spektrum völlig identisch mit einer nach Beispiel 3 erhaltenen Probe.
Ausbeute: 4,57 g (16 % der Theorie).

Beispiel 25

4,9-Dihydro-4-[[4-[3-(dimethylamino)propyl]-1-piperidinyl]acetyl]-3-methyl-10H-thieno[3,4-b][1,5]-benzodiazepin-10-on

Hergestellt analog Beispiel 1 aus 4-(Chloracetyl)-4,9-dihydro-3-methyl-10H-thieno[3,4-b][1,5]-benzodiazepin-10-on und 4-[3-(Dimethylamino)propyl]piperidin in einer Ausbeute von 27 % der Theorie.
Farblose Kristalle vom Fp. 196-197 °C (Essigester).

Beispiel 26

4-[[4-[3-(Diethylamino)propyl]-1-piperidinyl]acetyl]-4,9-dihydro-3-methyl-10H-thieno[3,4-b][1,5]-benzodiazepin-10-on

Hergestellt analog Beispiel 1 aus 4-(Chloracetyl)-4,9-dihydro-3-methyl-10H-thieno[3,4-b][1,5]-benzodiazepin-10-on und 4-[3-(Diethylamino)propyl]piperidin in einer Ausbeute von 32 % der Thorie.
Farblose Kristalle vom Fp. 209-210 °C (t. Butylmethylether).

Beispiel 27

4,9-Dihydro-3-methyl-4-[[4-[3-(1-piperidinyl)propyl]-1-piperidinyl]acetyl]-10H-thieno[3,4-b][1,5]benzodiazepin-10-on

Hergestellt analog Beispiel 1 aus 4-(Chloracetyl)-4,9-dihydro-3-methyl-10H-thieno[3,4-b][1,5]-benzodiazepin-10-on und 4-[4-(1-Piperidinyl)propyl]piperidin in einer Ausbeute von 46 % der Theorie.
Farblose Kristalle vom Fp. 214-215 °C (Essigester).

Beispiel 28

11-[4-[3-[(Diethylamino)propyl]-1-piperidinyl]acetyl]-6,11-dihydro-5H-pyrido[2,3-b][1,5]benzodiazepin-5-on

Hergestellt analog Beispiel 1 aus 11-(Chloracetyl)-6,11-dihydro-5H-pyrido[2,3-b][1,5]benzodiazepin-5-on und 4-[3-(Diethylamino)propyl]piperidin in einer Ausbeute von 24 % der Theorie.
Farblose Kristalle vom Fp. 168-169 °C (Essigester).

Beispiel 29

4,9-Dihydro-3-methyl-4-[[4-[4-(1-piperidinyl)butyl]-1-piperidinyl]acetyl]-10H-thieno[3,4-b][1,5]benzodiazepin-10-on

Hergestellt analog Beispiel 1 aus 4-(Chloracetyl)-4,9-dihydro-3-methyl-10H-thieno[3,4-b][1,5]-benzodiazepin-10-on und 4-[4-(1-Piperidinyl)butyl]piperidin in einer Ausbeute von 50 % der Theorie.
Farblose Kristalle vom Fp. 195-197 °C (Essigsäureethylester).

Beispiel 30

11-[[4-[4-(Diethylamino)butyl]-1-piperidinyl]acetyl]-5,11-dihydro-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 4-[4-(Diethylamino)butyl]piperidin in einer Ausbeute von 43 % der Theorie.
Farblose Kristalle vom Fp. 155-156 °C.

Beispiel 31

5,11-Dihydro-11-[[4-[3-(1-methyl-2-pyrrolidinyl)propyl]-1-piperidinyl]acetyl]-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 4-[3-(1-methyl-2-pyrrolidinyl)propyl]piperidin in einer Ausbeute von 32 % der Theorie. Farblose Kristalle vom Fp. 207-209 °C (Acetonitril).

Beispiel 32

11-[[4-[5-(Diethylamino)pentyl]-1-piperidinyl]acetyl]-5,11-dihydro-6H-pyrido[2,3-b][1,4]benzodiazepin-6-on

Hergestellt analog Beispiel 1 aus 11-(Chloracetyl)-5,11-dihydro-6H-pyrido[2,3-b][1,4]benzodiazepin-6-on und 4-[5-(Diethylamino)pentyl]piperidin in einer Ausbeute von 21 % der Theorie. Farblose Kristalle vom Fp. 137-138 °C (Essigsäureethylester).

Beispiel 33

trans-4,9-Dihydro-4-[[4-[3-[(4-hydroxycyclohexyl)methylamino]propyl]-1-piperidinyl]acetyl]-3-methyl-10H-thieno-[3,4-b][1,5]benzodiazepin-10-on

Hergestellt analog Beispiel 1 aus 4-(Chloracetyl)-4,9-dihydro-3-methyl-10H-thieno[3,4-b][1,5]-benzodiazepin-6-on und trans-4-[3-[(4-hydroxycyclohexyl)methylamino]propyl]-piperidin in einer Ausbeute von 10 % der Theorie. Farblose Kristalle vom Fp. 150-151 °C (Essigsäureethylester/Dichlormethan 3:1 v/v).

Beispiel 34

4,9-Dihydro-3-methyl-4-[[4-[3-(4-methyl-1-piperazinyl)-propyl]-1-piperidinyl]acetyl]-10H-thieno[3,4-b][1,5]-benzodiazepin-10-on

Hergestellt analog Beispiel 1 aus 4-(Chloracetyl)-4,9-dihydro-3-Methyl-10H-thieno[3,4-b][1,5]-benzodiazepin-10-on und 4-[3-(4-methyl-1-piperazinyl)propyl]piperidin in einer Ausbeute von 40 % der Theorie. Farblose Kristalle vom Fp. 217-218 °C (Essigsäureethylester).

Beispiel 35

11-[[2-[2-[2-(Diethylamino)ethoxy]ethyl]-1-piperidinyl]acetyl]-5,11-dihydro-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 2-[2-[2-(Diethylamino)ethoxy]ethyl]piperidin ($Sdp._{0,4\ mmHg}$ 95-99 °C) in einer Ausbeute von 44 % der Theorie. Farblose Kristalle vom Fp. 102-104 °C (umkristallisiert aus Diisopropylether und Cyclohexan).

Beispiel 36

11-[[4-[2-[2-(Diethylamino)ethoxy]ethyl]-1-piperidinyl]-acetyl]-5,11-dihydro-6H-pyrido[2,3-b][1,4]-benzodiazepin-6-on

Hergestellt analog Beispiel 1 aus 11-(Chloracetyl)-5,11-dihydro-6H-pyrido[2,3-b][1,4]benzodiazepin-6-on und 4-[2-[2-(Diethylamino)ethoxy]ethyl]piperidin ($Sdp._{0,009\ mmHg}$ 96-102 °C) in einer Ausbeute von 46 % der Theorie. Farblose Kristalle vom Fp. 130-131 °C (Acetonitril).

Beispiel 37

9-Chlor-11-[[4-[2-[2-(diethylamino)ethoxy]ethyl]-1-piperidinyl]acetyl]-5,11-dihydro-6H-pyrido[2,3-b][1,4]-benzodiazepin-6-on

Hergestellt analog Beispiel 1 aus 9-Chlor-11-(chloracetyl)-5,11-dihydro-6H-pyrido[2,3-b][1,4]-benzodiazepin-6-on und 4-[2-[2-(Diethylamino)ethoxy]ethyl]piperidin in einer Ausbeute von 35 % der Theorie. Farblose Kristalle vom Fp. 165,5-166,6 °C (Acetonitril/n-Propanol 3:1 v/v).

Beispiel 38

3-Chlor-4-[[4-[4-(diethylamino)butyl]-1-piperidinyl]acetyl]-1-methyl-1,4,9,10-tetrahydropyrrolo[3,2-b][1,5]-benzodiazepin-10-on

Hergestellt analog Beispiel 1 aus 3-Chlor-4-(chloracetyl)-1-methyl-1,4,9,10-tetrahydropyrrolo[3,2-b][1,5]-benzodiazepin-10-on und 4-[4-(Diethylamino)butyl]piperidin in einer Ausbeute von 34 % der Theorie. Farblose Kristalle vom Fp. 158-160 °C.

Beispiel 39

4-[[4-[4-(Diethylamino)butyl]-1-piperidinyl]acetyl]-1-methyl-1,4,9,10-tetrahydropyrrolo[3,2-b][1,5]-benzodiazepin-10-on

3,957 g (8,14 mMol) 3-Chlor-4-[[4-[4-(diethylamino)butyl]-1-piperidinyl]acetyl]-1-methyl-1,4,9,10-tetrahydropyrrolo-[3,2-b][1,5]benzodiazepin-10-on wurden in 350 ml heißem Ethanol gelöst und nach Zusatz von 3 g Palladium auf Tierkohle (20proz.) 20 Stunden bei einem Wasserstoffdruck von 50 bar und einer Temperatur von 40 °C hydriert. Man filtrierte vom Katalysator ab, engte das Filtrat im Vakuum ein, nahm das kristalline Hydrochlorid in 20 ml Wasser auf, stellte die erhaltene Lösung natronalkalisch und extrahierte erschöpfend mit Dichlormethan. Die vereinigten Extrakte wurden über Natriumsulfat getrocknet und eingedampft und der verbleibende Rückstand umkristallisiert. Man erhielt 1 g (26,3 % der Theorie) an farblosen Kristallen vom Fp. 196-198 °C.

Beispiel 40

4-[[4-[4-(Diethylamino)butyl]-1-piperidinyl]acetyl]-1-methyl-1,4,9,10-tetrahydropyrrolo[3,2-b][1,5]-benzodiazepin-10-on

4,715 g (9,7 mMol) 3-Chlor-4-[[4-[4-(diethylamino)butyl]-1-piperidinyl]acetyl]-1-methyl-1,4,9,10-tetrahydropyrrolo-[3,2-b][1,5]benzodiazepin-10-on wurden in einem Gemisch aus 5 ml 85%iger Ameisensäure und 25 ml Dimethylformamid gelöst und nach Zusatz von 0,5 g 10%igem Palladium/Aktivkohle 3 Stunden unter Rückfluß gekocht. Man gab 7,0 ml Ameisensäure zu, kochte weiter 6 Stunden unter Rückfluß und erhitzte nach Versetzen mit weiteren 4,0 ml Ameisensäure und 0,8 g 10%igem Palladium/Aktivkohle abschließend nochmals 8 Stunden unter Rückfluß. Die Mischung wurde heiß filtriert, das Filtrat im Vakuum eingedampft und der Rückstand säulenchromatographisch (Kieselgel; Dichlormethan/Essigsäureethylester/Methanol/konz. Ammoniak 3,5:1,5:0,46:0,06 v/v) gereinigt. Man erhielt 1,45 g (33 % der Theorie) an farblosen Kristallen vom Fp. 196-198 °C (Acetonitril), nach Dünnschichtchromatogramm und IR-, UV- und $^1$H-NMR-Spektren identisch mit einem nach Beispiel 39 erhaltenen Präparat.

Beispiel 41

4-[[4-[4-(Diethylamino)butyl]-1-piperidinyl]acetyl]-1-methyl-1,4,9,10-tetrahydropyrrolo[3,2-b][1,5]-benzodiazepin-10-on

Die Mischung aus 4,86 g (0,01 Mol) 3-Chlor-4-[[4-[4-(diethylamino)butyl]-1-piperidinyl]acetyl]-1-methyl-1,4,9,10-tetrahydropyrrolo[3,2-b][1,5]benzodiazepin-10-on, 83,3 mg (0,001 Mol) 2:1-Tris(o-tolyl)-phosphin-Palladiumacetat-Katalysator, 2,025 g (0,044 Mol) Ameisensäure und 5,77 g (0,057 Mol)Triethylamin in 200 ml Tetrahydrofuran wurde unter Stickstoff-Atmosphäre im Autoklaven 40 Stunden auf 100 °C erhitzt. Die Mischung wurde filtriert und im Vakuum eingedampft, der Rückstand natronalkalisch gestellt und erschöpfend mit Dichlormethan extrahiert. Die getrockneten und eingedampften organischen Phasen wurden wie in Beispiel 40 säulenchromatographisch gereinigt. Man erhielt 1,76 g (3 % der Theorie) an farblosen Kristallen vom Fp. 196-198 °C (Acetonitril), nach Dünnschichtchromatogramm und IR-Spektrum identisch mit einer nach Beispiel 39 erhaltenen Probe.

Im folgenden wird die Herstellung pharmazeutischer Anwendungsformen anhand einiger Beispiele beschrieben:

Beispiel I

Tabletten mit 5 mg 4,9-Dihydro-3-methyl-4-[[4-[3-(1-piperidinyl)propyl]-1-piperidinyl]acetyl]-10H-thieno[3,4-b][1,5]-benzodiazepin-10-on

| Zusammensetzung: | |
|---|---|
| 1 Tablette enthält: | |
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

| | |
|---|---|
| Tablettengewicht: | 220 mg |
| Stempel: | 9 mm |

Beispiel II

Dragées mit 5 mg 4,9-Dihydro-3-methyl-4-[[4-[3-(1-piperidinyl)propyl]-1-piperidinyl]acetyl]-10H-thieno[3,4-b]-[1,5]-benzodiazepin-10-on

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.
Dragéegewicht: 300 mg

Beispiel III

Ampullen mit 10 mg 4,9-Dihydro-3-methyl-4-[[4-[3-(1-piperidinyl)propyl]-1-piperidinyl]acetyl]-10H-thieno[3,4-b][1,5]-benzodiazepin-10-on

| Zusammensetzung: | |
|---|---|
| 1 Ampulle enthält: | |
| Wirkstoff | 10,0 mg |
| Natriumchlorid | 8,0 mg |
| Dest. Wasser | ad 1 ml |

Herstellungsverfahren:

Die Wirksubstanz und Natriumchlorid werden in dest. Wasser gelöst und anschließend auf das gegebene Volumen aufgefüllt. Die Lösung wird sterilfiltriert und in 1 ml-Ampullen abgefüllt.
Sterilisation: 20 Minuten bei 120° C.

Beispiel IV

Suppositorien mit 20 mg 4,9-Dihydro-3-methyl-4-[[4-[3-(1-piperidinyl)propyl]-1-piperidinyl]acetyl]-10H-thieno-[3,4-b][1,5]benzodiazepin-10-on

| Zusammensetzung: | |
|---|---|
| 1 Zäpfchen enthält: | |
| Wirkstoff | 20,0 mg |
| Zäpfchenmasse (z.B. Witepsol W 45®) | 1 680,0 mg |
| | 1 700,0 mg |

Herstellungsverfahren:

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus.
Zäpfchengewicht 1,7 g

Beispiel V

Tropfen mit 4,9-Dihydro-3-methyl-4-[[4-[3-(1-piperidinyl)-propyl]-1-piperidinyl]acetyl]-10H-thieno[3,4-b][1,5]-benzodiazepin-10-on

| Zusammensetzung: | |
|---|---|
| 100 ml Tropflösung enthalten: | |
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser | ad 100,0 ml |

Herstellungsverfahren:

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser aus 100 ml aufgefüllt und schwebeteilchenfrei filtriert.

## Ansprüche

1.) Kondensierte Diazepinone der allgemeinen Formel

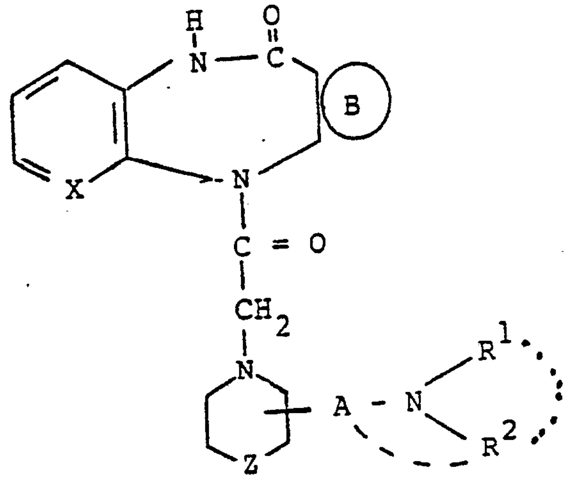

(I),

in der

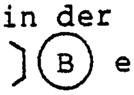 einen der zweiwertigen Reste

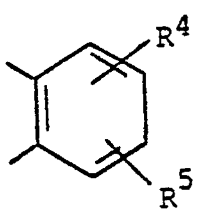 ,

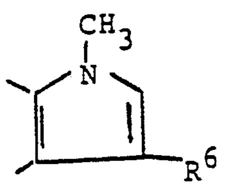 ,

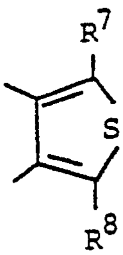 ,

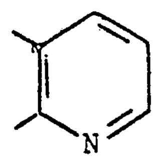

(S) (T) (U) (V)

darstellt und

A, X, Z, $R^1$, $R^2$ und $R^4$ bis $R^8$ die folgenden Bedeutungen besitzen:

X ist eine =CH-Gruppe oder ein Stickstoffatom,

A ist ein geradkettiger oder verzweigter, gesättigter Alkylenrest mit 3 bis 7 Kohlenstoffatomen, der auch durch ein Sauerstoff- oder Schwefelatom oder die Gruppe >$NR^3$ unterbrochen sein kann, worin $R^3$ eine Alkylgruppe mit 1 bis 3 Kohlenstoffatomen darstellt,

Z ist eine Einfachbindung, ein Sauerstoff- oder Schwefelatom, die Methylen- oder 1,2-Ethylengruppe,

$R^1$ ist ein verzweigter oder unverzweigter Alkylrest mit 1 bis 7 Kohlenstoffatomen, ein Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor- oder Bromatom und/oder eine Methyl-, Methoxy- oder Trifluormethylgruppe substituierte Benzoylgruppe,

$R^2$ bedeutet eine verzweigte oder unverzweigte Alkylgruppe mit 1 bis 6 Kohlenstoffatomen, $R^2$ kann aber auch ein Wasserstoffatom sein, wenn $R^1$ die vorerwähnte aliphatische Acylgruppe oder gegebenenfalls substituierte Benzoylgruppe darstellt,

$R^1$ und $R^2$ können aber auch zusammen mit dem dazwischenliegenden Stickstoffatom einen gesättigten, monocyclischen 5-, 6- oder 7-gliedrigen Ring bilden, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,

$R^2$ kann aber auch mit einem Kohlenstoffatom der A-Kette in der Weise verknüpft sein, daß zusammen mit

der Gruppe $NR^1$ ein gesättigter 5-, 6- oder 7-gliedriger heterocyclischer Ring entsteht,
$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,
$R^6$ ist ein Wasserstoff- oder Chloratom oder eine Methylgruppe,
$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten,
ihre diastereomere resp. enantiomere Formen und ihre physiologisch verträgliche Salze mit anorganischen oder organischen Säuren.

2.) Kondensierte Diazepinone der allgemeinen Formel I gemäß Anspruch 1, bei welchen die Gruppe

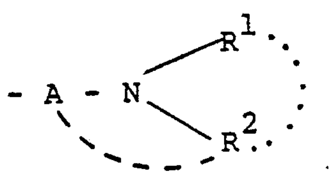

in der 3- oder 4-Stellung des gesättigten heterocyclischen Restes sitzt und
A einen geradkettigen oder verzweigten Alkylenrest mit 3 bis 5 Kohlenstoffatomen, der gegebenenfalls durch die Gruppe $>N-CH_3$ unterbrochen sein kann,
X ein Stickstoffatom oder die $=CH$-Gruppe,
]Ⓑ den zweiwertigen Rest (S) oder (U), worin $R^4$ und $R^5$ Wasserstoffatome, $R^7$ und $R^8$ Wasserstoffatome oder einer der Reste $R^4$, $R^5$, $R^7$ oder $R^8$ eine Methylgruppe darstellen,
$R^1$ und $R^2$, die gleich oder voneinander verschieden sein können, Alkylreste mit 1 bis 3 Kohlenstoffatomen oder zusammen mit dem dazwischenliegenden Stickstoffatom den 1-Piperidinylrest oder $R^2$ zusammen mit dem entsprechenden Kohlenstoffatom der Gruppe -A- den 4-Piperidinylrest der Formel

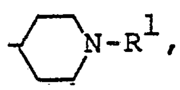

wobei $R^1$ eine niedere Alkylgruppe mit 1 bis 3 Kohlenstoffatomen darstellt und Z die Methylengruppe bedeuten, ihre diastereomere resp. enantiomere Formen und ihre physiologisch verträgliche Salze mit anorganischen oder organischen Säuren.

3.) 5,11-Dihydro-11-[[4-[4-(1-piperidinyl)butyl]-1-piperidinyl]acetyl]-6H-pyrido[2,3-b][1,4]benzodiazepin-6-on und seine physiologisch verträglichen Salze mit anorganischen oder organischen Säuren.

4.) 5,11-Dihydro-11-[[4-[3-(1-methyl-4-piperidinyl)propyl]-1-piperidinyl]acetyl]-6H-pyrido[2,3-b][1,4]-benzodiazepin-6-on und seine physiologisch verträglichen Salze mit anorganischen oder organischen Säuren.

5.) 5,11-Dihydro-11-[[4-[3-(1-piperidinyl)propyl]-1-piperidinyl]acetyl]-6H-pyrido[2,3-b][1,4]benzodiazepin-6-on und seine physiologisch verträglichen Salze mit anorganischen oder organischen Säuren.

6.) 4,9-Dihydro-3-methyl-4-[[4-[3-(1-piperidinyl)propyl]-1-piperidinyl]acetyl]-10H-thieno[3,4-b][1,5]-benzodiazepin-10-on und seine physiologisch verträglichen Salze mit anorganischen oder organischen Säuren.

7.) Arzneimittelzubereitungen enthaltend mindestens ein kondensiertes Diazepinon gemäß Anspruch 1 bis 6 neben üblichen Träger- und/oder Hilfsstoffen.

8.) Verfahren zur Herstellung von neuen kondensierten Diazepinonen der allgemeinen Formel I

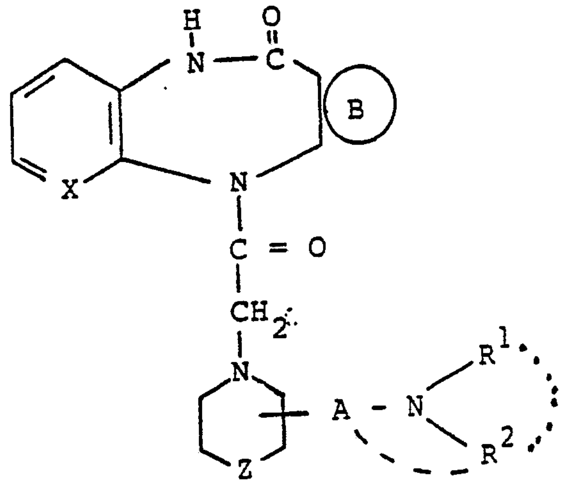

(I),

in der

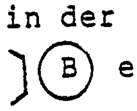 einen der zweiwertigen Reste

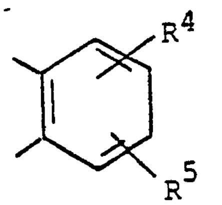 ,

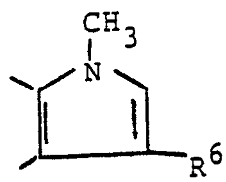 ,

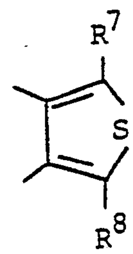 ,

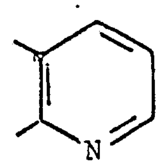

(S) (T) (U) (V)

darstellt und

A, X, Z, $R^1$, $R^2$ und $R^4$ bis $R^8$ die folgenden Bedeutungen besitzen:

X ist eine =CH-Gruppe oder ein Stickstoffatom, A ist ein geradkettiger oder verzweigter, gesättigter Alkylenrest mit 3 bis 7 Kohlenstoffatomen, der auch durch ein Sauerstoff- oder Schwefelatom oder die Gruppe $>NR^3$ unterbrochen sein kann, worin $R^3$ eine Alkylgruppe mit 1 bis 3 Kohlenstoffatomen darstellt,

Z ist eine Einfachbindung, ein Sauerstoff- oder Schwefelatom, die Methylen- oder 1,2-Ethylengruppe,

$R^1$ ist ein verzweigter oder unverzweigter Alkylrest mit 1 bis 7 Kohlenstoffatomen, ein Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor- oder Bromatom und/oder eine Methyl-, Methoxy- oder Trifluormethylgruppe substituierte Benzoylgruppe,

$R^2$ bedeutet eine verzweigte oder unverzweigte Alkylgruppe mit 1 bis 6 Kohlenstoffatomen, $R^2$ kann aber auch ein Wasserstoffatom sein, wenn $R^1$ die vorerwähnte aliphatische Acylgruppe oder gegebenenfalls substituierte Benzoylgruppe darstellt,

$R^1$ und $R^2$ können aber auch zusammen mit dem dazwischenliegenden Stickstoffatom einen gesättigten, monocyclischen 5-, 6- oder 7-gliedrigen Ring bilden, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,

$R^2$ kann aber auch mit einem Kohlenstoffatom der A-Kette in der Weise verknüpft sein, daß zusammen mit der Gruppe $NR^1$ ein gesättigter 5-, 6- oder 7-gliedriger heterocyclischer Ring entsteht,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,

$R^7$ und $R^8$, die gleich oder voneinander verschieden sein können, bedeuten Wasserstoffatome oder

Alkylgruppen mit 1 bis 4 Kohlenstoffatomen, $R^8$ kann aber auch zusätzlich ein Halogenatom bedeuten und von ihren diastereomeren resp. enantiomeren Formen und ihren physiologisch verträglichen Salzen mit anorganischen oder organischen Säuren, dadurch gekennzeichnet, daß
a.) zur Herstellung von basisch substituierten kondensierten Diazepinonen der allgemeinen Formel

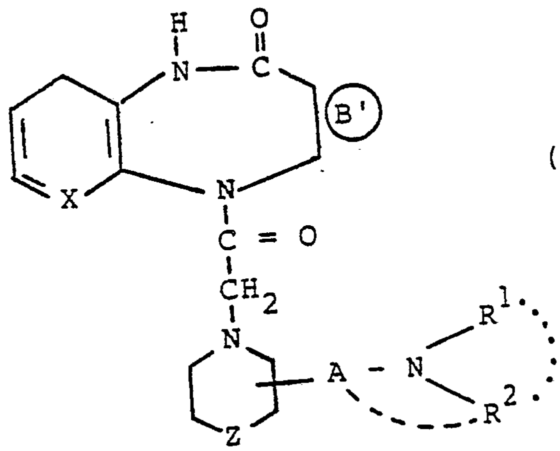

(Ia),

in der
X, Z, A und $R^1$ und $R^2$ wie oben angegeben definiert sind und 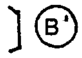 einen der zweiwertigen Reste (S), (U), (V) oder (T′)

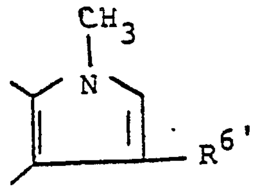

(T′)

darstellt, worin $R^{6'}$ ein Chloratom oder eine Methylgruppe ist, Halogenacylverbindungen der allgmemeinen Formel II,

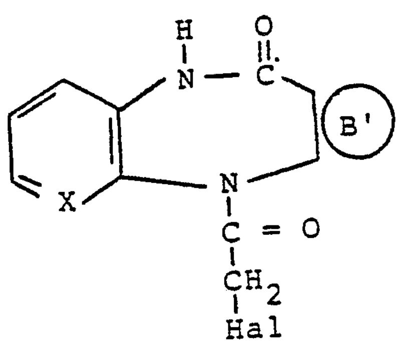

(II),

in der X und 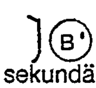 die vorstehend genannten Bedeutungen haben und Hal ein Chlor-, Brom- oder Iodatom ist, mit sekundären Aminen der allgemeinen Formel III

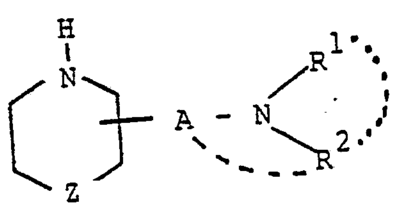

(III)

mit den für A, Z, $R^1$ und $R^2$ genannten Bedeutungen, in einem Lösungsmittel bei Temperaturen zwischen -10 °C und der Siedetemperatur des Reaktionsgemisches, vorzugsweise in Gegenwart einer Hilfsbase oder eines Überschußes des Amins der allgemeinen Formel III, umgesetzt werden, oder

b.) zur Herstellung von basisch substituierten Diazepinonen der allgemeinen Formel Ia, Diazepinone der allgemeinen Formel IV

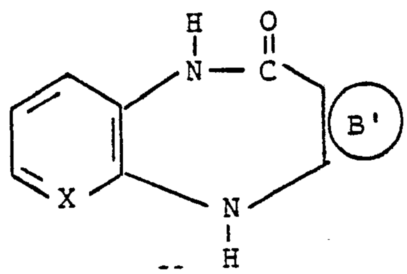

(IV),

worin X und ] (B') die oben angegebenen Bedeutungen haben, mit Carbonsäurederivaten der allgemeinen Formel V,

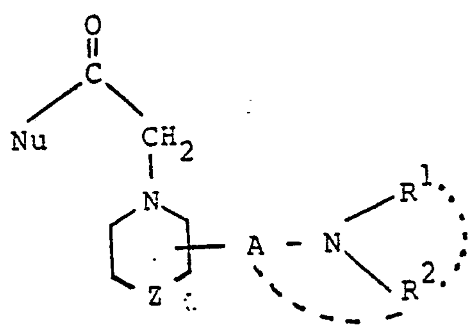

(V),

worin Z, A, $R^1$ und $R^2$ die oben angegebenen Bedeutungen haben und Nu eine nucleofuge Gruppe bzw. Abgangsgruppe darstellt, in einem inerten Lösungsmittel bei Temperaturen zwischen -25 °C und 130 °C umgesetzt werden oder

c.) zur Herstellung von unter die allgemeine Formel I fallenden Pyrrolo-kondensierten Diazepinonen der allgemeinen Formel Ib

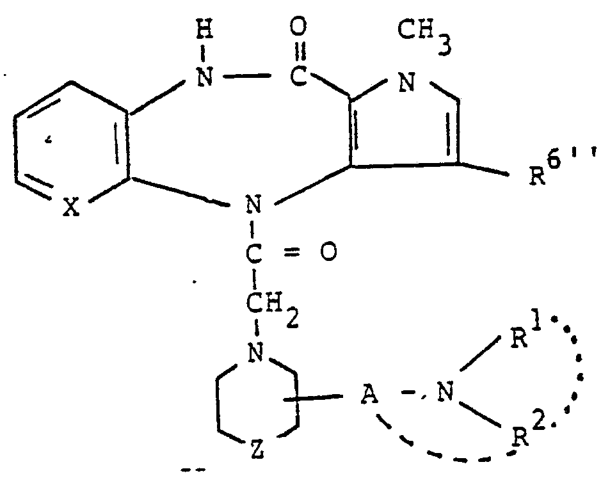

(Ib),

worin

X, Z, A, $R^1$ und $R^2$ die eingangs erwähnten Bedeutungen haben und $R^{6''}$ ein Wasserstoffatom ist, Verbindungen der allgemeinen Formel Ia

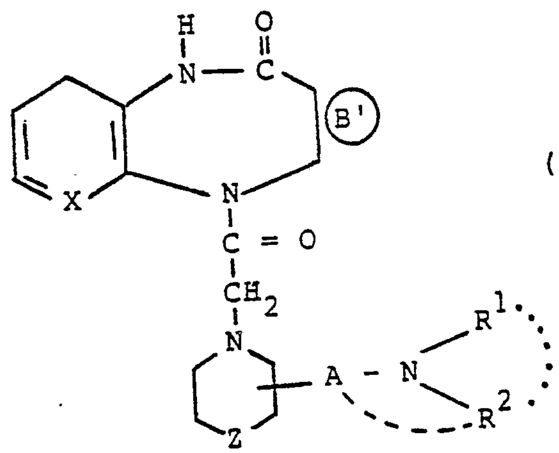

(Ia),

worin X, Z, A und $R^1$ und $R^2$ wie oben angegeben definiert sind und ] (B') den zweiwertigen Rest (T')

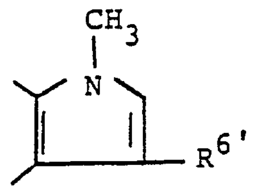

(T')

darstellt, worin $R^{6'}$ ein Chloratom ist, in Anwesenheit eines Lösungsmittels bei Temperaturen von 0° bis 130° C und in Gegenwart von Katalysatoren der Metalle der VIII. Nebengruppe des Periodensystems bei Wasserstoffdrucken von 1 bis 300 bar, oder, sofern der Wasserstoff durch Ameisensäure oder Triethylammoniumformiat in Gegenwart von Triethylamin ersetzt wird, drucklos einer Hydrogenolyse unterworfen werden,
die erhaltenen Verbindungen der allgemeinen Formel I gegebenenfalls anschließend in ihre diastereomeren resp. enantiomeren Formen aufgetrennt und/oder die erhaltenen Salze in die freien Basen und/oder die erhaltenen freien Basen in ihre physiologisch verträglichen Salze mit anorganischen oder organischen Säuren übergeführt werden.

9.) Verfahren gemäß Anspruch 8b, dadurch gekennzeichnet, daß als reaktive Carbonsäurederivate der allgemeinen Formel V deren Säurehalogenide, -ester, -anhydride oder gemischte Anhydride, insbesondere deren gemischte Anhydride mit starken Mineralsäuren verwendet werden, die Reaktion in Gegenwart von säurebindenden Mitteln durchgeführt wird und als inerte Lösungsmittel chlorierte aliphatische Kohlenwasserstoffe, offenkettige oder cyclische Ether, aromatische Kohlenwasser stoffe oder polare aprotische Lösungsmittel oder Gemische dieser Lösungsmittel eingesetzt werden.

10.) Verfahren gemäß Anspruch 8c, dadurch gekennzeichnet, daß die Hydrogenolyse

a.) mit Palladium auf Tierkohle oder auf Bariumsulfat, mit Raney-Nickel oder Raney-Cobalt als Katalysatoren und in Gegenwart von Alkoholen, Ethern, Carbonsäuren oder tertiären Aminen als Lösungsmittel oder

b.) mit Ameisensäure, gegebenenfalls in Gegenwart von Dimethylformamid als Lösungsmittel und von Palladium auf Kohle als Katalysator bei Temperaturen zwischen 70 und 100° C, oder

c.) mit Triethylammoniumformiat in Gegenwart überschüssigen Triethylamins und von Palladium auf Tierkohle oder von Palladiumacetat und Triarylphosphinen bei Temperaturen zwischen 40 und 110° C durchgeführt wird.

Patentansprüche für folgende Vertragsstaaten: ES, GR.

1.) Verfahren zur Herstellung von neuen kondensierten Diazepinonen der allgemeinen Formel I

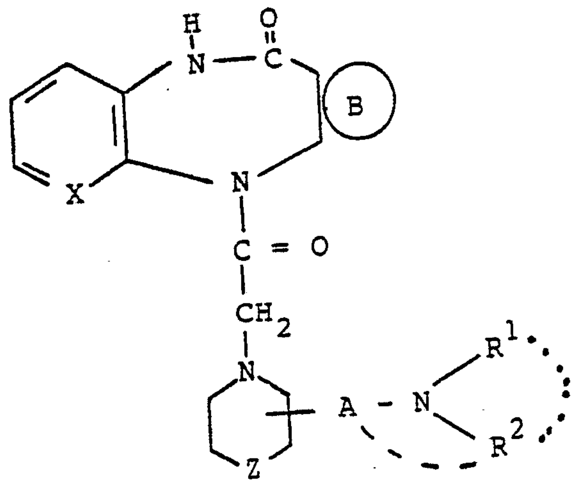

(I),

in der

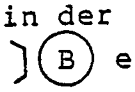 einen der zweiwertigen Reste

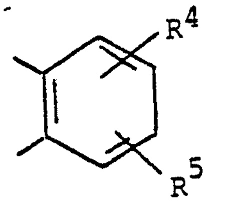 , 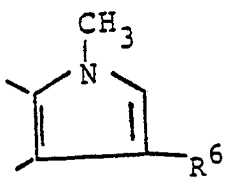 , 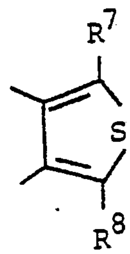 , 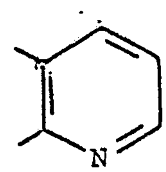

(S) (T) (U) (V)

darstellt und

A, X, Z, $R^1$, $R^2$ und $R^4$ bis $R^8$ die folgenden Bedeutungen besitzen:

X ist eine =CH-Gruppe oder ein Stickstoffatom,

A ist ein geradkettiger oder verzweigter, gesättigter Alkylenrest mit 3 bis 7 Kohlenstoffatomen, der auch durch ein Sauerstoff- oder Schwefelatom oder die Gruppe $>NR^3$ unterbrochen sein kann, worin $R^3$ eine Alkylgruppe mit 1 bis 3 Kohlenstoffatomen darstellt,

Z ist eine Einfachbindung, ein Sauerstoff- oder Schwefelatom, die Methylen- oder 1,2-Ethylengruppe,

$R^1$ ist ein verzweigter oder unverzweigter Alkylrest mit 1 bis 7 Kohlenstoffatomen, ein Cycloalkyl- oder (Cycloalkyl)alkylrest mit insgesamt bis zu 8 Kohlenstoffatomen, eine gegebenenfalls mit einem Fluor-, Chlor- oder Bromatom und/oder einer Methyl-, Methoxy- oder Trifluormethylgruppe substituierte Aralkylgruppe mit bis zu 9 Kohlenstoffatomen, eine aliphatische Acylgruppe mit bis zu 7 Kohlenstoffatomen oder eine gegebenenfalls durch ein Fluor-, Chlor- oder Bromatom und/oder eine Methyl-, Methoxy- oder Trifluormethylgruppe substituierte Benzoylgruppe,

$R^2$ bedeutet eine verzweigte oder unverzweigte Alkylgruppe mit 1 bis 6 Kohlenstoffatomen, $R^2$ kann aber auch ein Wasserstoffatom sein, wenn $R^1$ die vorerwähnte aliphatische Acylgruppe oder gegebenenfalls substituierte Benzoylgruppe darstellt,

$R^1$ und $R^2$ können aber auch zusammen mit dem dazwischenliegenden Stickstoffatom einen gesättigten, monocyclischen 5-, 6- oder 7-gliedrigen Ring bilden, der gegebenenfalls noch durch eine Aminocarbonyl-, Dimethylaminocarbonyl- oder Diethylaminocarbonylgruppe substituiert und/oder durch ein Sauerstoffatom unterbrochen sein kann,

$R^2$ kann aber auch mit einem Kohlenstoffatom der A-Kette in der Weise verknüpft sein, daß zusammen mit der Gruppe $NR^1$ ein gesättigter 5-, 6- oder 7-gliedriger heterocyclischer Ring entsteht,

$R^4$ und $R^5$, die gleich oder voneinander verschieden sein können, bedeuten ein Wasserstoff-, Fluor-, Chlor- oder Bromatom oder eine Alkylgruppe mit 1 bis 4 Kohlenstoffatomen,

$R^6$ ist ein Wasserstoff- oder Chloratom oder eine Methylgruppe,
$R^7$ und $R^8$, die gleich oder voneinander verschieden sein können, bedeuten Wasserstoffatome oder Alkylgruppen mit 1 bis 4 Kohlenstoffatomen, $R^8$ kann aber auch zusätzlich ein Halogenatom bedeuten
und von ihren diastereomeren resp. enantiomeren Formen und ihren physiologisch verträglichen Salzen mit anorganischen oder organischen Säuren, dadurch gekennzeichnet, daß
a.) zur herstellung von basisch substituierten kondensierten Diazepinonen der allgemeinen Formel

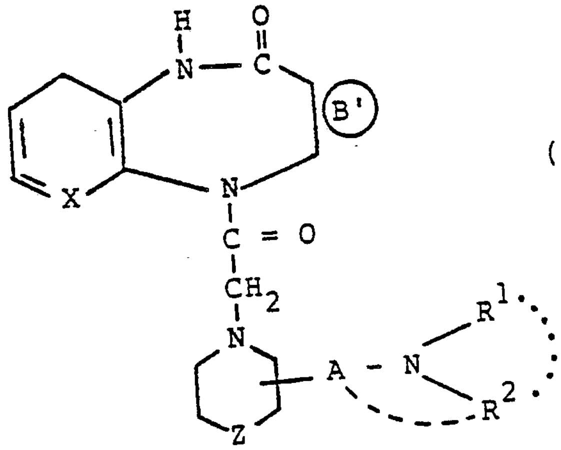

(Ia),

in der
X, Z, A und $R^1$ und $R^2$ wie oben angegeben definiert sind und 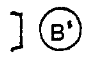 einen der zweiwertigen Reste (S), (U), (V) oder (T′)

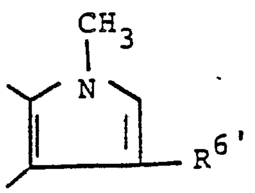

(T')

darstellt, worin $R^{6'}$ ein Chloratom oder eine Methylgruppe ist, Halogenacylverbindungen der allgmemeinen Formel II,

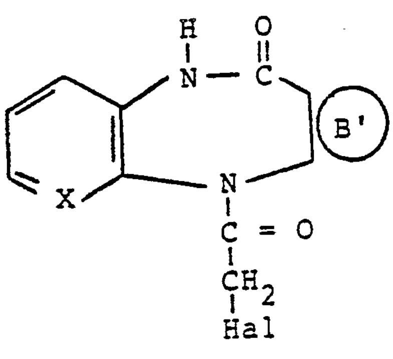

(II),

in der X und 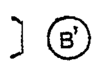 die vorstehend genannten Bedeutungen haben und Hal ein Chlor-, Brom- oder lodatom ist, mit sekundären Aminen der allgemeinen Formel III

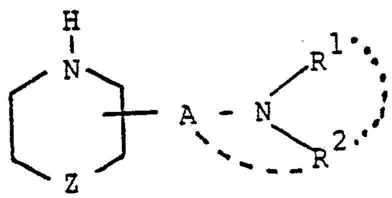

(III)

mit den für A, Z, $R^1$ und $R^2$ genannten Bedeutungen, in einem Lösungsmittel bei Temperaturen zwischen -10° C und der Siedetemperatur des Reaktionsgemisches, vorzugsweise in Gegenwart einer Hilfsbase oder eines Überschußes des Amins der allgemeinen Formel III, umgesetzt werden, oder

b.) zur Herstellung von basisch substituierten Diazepinonen der allgemeinen Formel Ia, Diazepinone der allgemeinen Formel IV

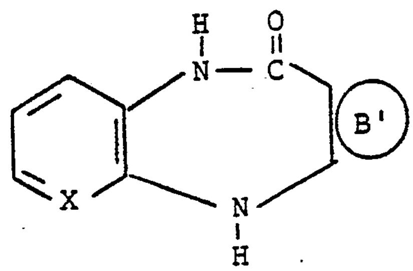

(IV),

worin X und 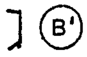 die oben angegebenen Bedeutungen haben, mit Carbonsäurederivaten der allgemeinen Formel V,

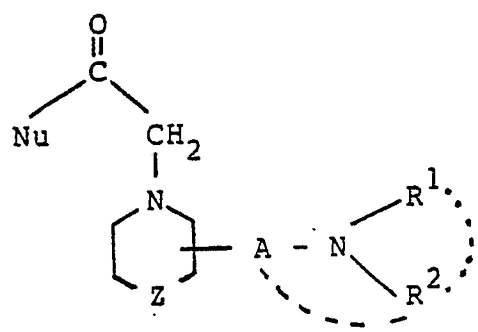

(V),

worin Z, A, $R^1$ und $R^2$ die oben angegebenen Bedeutungen haben und Nu eine nucleofuge Gruppe bzw. Abgangsgruppe darstellt, in einem inerten Lösungsmittel bei Temperaturen zwischen -25° C und 130° C umgesetzt werden oder

c.) zur Herstellung von unter die allgemeine Formel I fallenden Pyrrolo-kondensierten Diazepinonen der allgemeinen Formel Ib

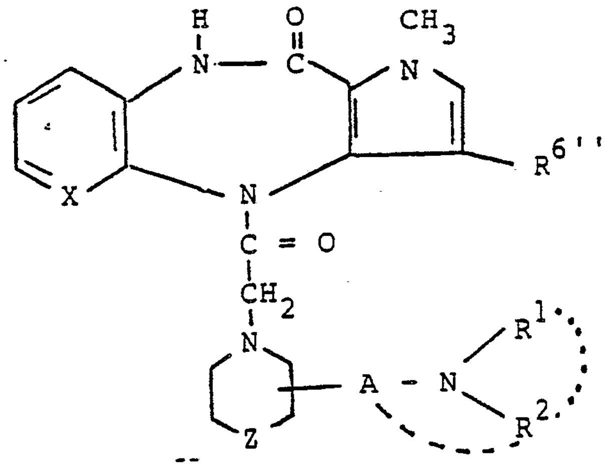

(Ib),

worin
X, Z, A, $R^1$ und $R^2$ die eingangs erwähnten Bedeutungen haben und $R^{6''}$ ein Wasserstoffatom ist, Verbindungen der allgemeinen Formel Ia

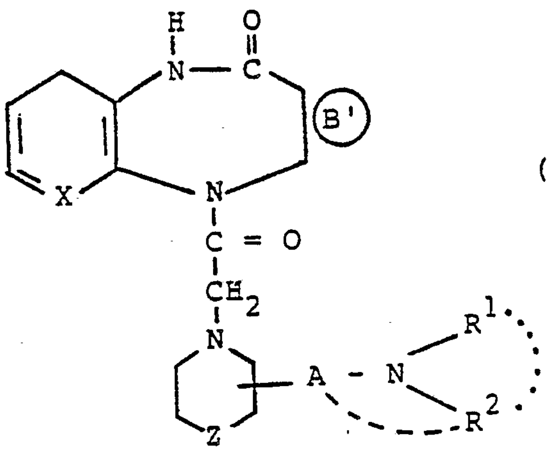

(Ia),

worin X, Z, A und $R^1$ und $R^2$ wie oben angegeben definiert sind und $B'$ den zweiwertigen Rest (T')

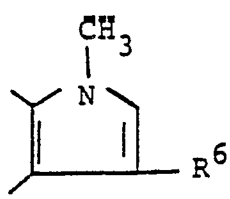

(T')

darstellt, worin $R^{6'}$ ein Chloratom ist, in Anwesenheit eines Lösungsmittels bei Temperaturen von 0° bis 130°C und in Gegenwart von Katalysatoren der Metalle der VIII. Nebengruppe des Periodensystems bei Wasserstoffdrucken von 1 bis 300 bar, oder, sofern der Wasserstoff durch Ameisensäure oder Triethylammoniumformiat in Gegenwart von Triethylamin ersetzt wird, drucklos einer Hydrogenolyse unterworfen werden,
die erhaltenen Verbindungen der allgemeinen Formel I gegebenenfalls anschließend in ihre diastereomeren resp. enantiomeren Formen aufgetrennt und/oder die erhaltenen Salze in die freien Basen und/oder die erhaltenen freien Basen in ihre physiologisch verträglichen Salze mit anorganischen oder organischen Säuren übergeführt werden.

2.) Verfahren gemäß Anspruch 1b, dadurch gekennzeichnet, daß als reaktive Carbonsäurederivate der allgemeinen Formel V deren Säurehalogenide, -ester, -anhydride oder gemischte Anhydride, insbesondere deren gemischte Anhydride mit starken Mineralsäuren verwendet werden, die Reaktion in Gegenwart von

säurebindenden Mitteln durchgeführt wird und als inerte Lösungsmittel chlorierte aliphatische Kohlenwasserstoffe, offenkettige oder cyclische Ether, aromatische Kohlenwasserstoffe oder polare aprotische Lösungsmittel oder Gemische dieser Lösungsmittel eingesetzt werden.

3.) Verfahren gemäß Anspruch 1c, dadurch gekennzeichnet, daß die Hydrogenolyse

a.) mit Palladium auf Tierkohle oder auf Bariumsulfat, mit Raney-Nickel oder Raney-Cobalt als Katalysatoren und in Gegenwart von Alkoholen, Ethern, Carbonsäuren oder tertiären Aminen als Lösungsmittel oder

b.) mit Ameisensäure, gegebenenfalls in Gegenwart von Dimethylformamid als Lösungsmittel und von Palladium auf Kohle als Katalysator bei Temperaturen zwischen 70 und 110 °C, oder

c.) mit Triethylammoniumformiat in Gegenwart überschüssigen Triethylamins und von Palladium auf Tierkohle oder von Palladiumacetat und Triarylphosphinen bei Temperaturen zwischen 40 und 110 °C durchgeführt wird.